# EUROPÄISCHE PATENTSCHRIFT

(11) **EP 3 185 793 B1**
(45) Veröffentlichungstag und Bekanntmachung des Hinweises auf die Patenterteilung: **09.10.2019**
(21) Anmeldenummer: 15756390.9
(22) Anmeldetag: 25.08.2015
(51) Int. Cl.: A61B 17/72

(54) **VERRIEGELUNGSMARKNAGEL ZUM EINBRINGEN IN DEN MARKRAUM EINES RÖHRENKNOCHENS**
INTRAMEDULLARY NAIL FOR LONG BONES
CLOU DE VERROUILLAGE CENTROMÉDULLAIRE À APPLIQUER DANS LA CAVITÉ MÉDULLAIRE D'UN OS LONG

(30) Priorität: 26.08.2014 DE 102014112213
(43) Veröffentlichungstag der Anmeldung: 05.07.2017
(73) Patentinhaber: OT Medizintechnik GmbH, 80639 München (DE)
(72) Erfinder: SCHREIBER, Ulrich, 80639 München (DE)
(74) Vertreter: Bobbert & Partner Patentanwälte PartmbB
(86) Internationale Anmeldenummer: PCT/EP2015/069425
(87) Internationale Veröffentlichungsnummer: WO 2016/030360

(56) Entgegenhaltungen:
- WO-A1-2011/002753
- WO-A1-2011/018778
- WO-A1-2013/120034
- JP-B2- 2 973 316
- US-A1- 2005 055 024
- US-A1- 2010 179 550
- US-A1- 2012 197 255

## Beschreibung

Die Erfindung bezieht sich auf einen Verriegelungsmarknagel zum Einbringen in den Markraum eines Röhrenknochens nach Anspruch 1.

Marknägel sind bekannte Hilfsmittel zur Versorgung von Brüchen langer Röhrenknochen. Sie werden in den Markraum eines frakturierten Knochens eingebracht, um die Knochenfraktur mechanisch zu überbrücken. Marknägel können als sogenannte Verriegelungsmarknägel ausgestaltet sein. Bei Letzteren dienen Verriegelungsschrauben dazu, die Verbindung zwischen Knochen und Verriegelungsmarknagel gegen Verschiebung zu sichern.

Bei bisher üblichen Verriegelungsmarknägeln werden die Verriegelungsschrauben in diskreten Öffnungen des Marknagels in einer vorbestimmten Stellung zum Marknagel im Knochen platziert. Das exakte Platzieren der Verriegelungsschrauben in dem im Röhrenknochen angeordneten Marknagel setzt eine große Erfahrung des Operateurs beim Einbringen des Marknagels in den Knochen voraus.

Aus der WO 2011/002753 A1 sind ein intramedullärer Nagel und ein überstehender Schraubverriegelungsmechanismus bekannt.

Aufgabe der vorliegenden Erfindung ist es, einen Verriegelungsmarknagel zum Einbringen in den Markraum eines Röhrenknochens zur Versorgung von Knochenfrakturen und/ oder zur Versteifung von Gelenken anzugeben.

Die erfindungsgemäße Aufgabe wird mit einem Verriegelungsmarknagel mit den Merkmalen des Anspruchs 1 gelöst.

Im Folgenden werden die Begriffe Verriegelungsmarknagel und Marknagel synonym verwendet.

Der erfindungsgemäße Verriegelungsmarknagel umfasst einen länglichen Schaft mit wenigstens einer Öffnung zur Aufnahme von wenigstens einer Verriegelungsvorrichtung und wenigstens einer Aufnahmeeinrichtung zur Aufnahme der Verriegelungsvorrichtung. Die Aufnahmeeinrichtung ist im Bereich der Öffnung zur Aufnahme von wenigstens einer Verriegelungsvorrichtung angeordnet. Weiterhin umfasst der Verriegelungsmarknagel wenigstens einen Stützkörper, welcher in Kontakt mit wenigstens einer Aufnahmeeinrichtung angeordnet ist.

Der erfindungsgemäße Verriegelungsmarknagel kann der Versorgung des Humerus, des Femur, des Sprunggelenks, des Kniegelenks oder jedes anderen Knochens oder Gelenks des menschlichen Körpers oder des Tieres (Pferde, Hunde, usw.) dienen.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Erfindungsgemäße Ausführungsformen jeder der vorgenannten Gegenstände können eines oder mehrere der im Folgenden genannten Merkmale in beliebiger Kombination aufweisen, sofern eine konkrete Kombination für den Fachmann nicht als offenkundig technisch unmöglich erkennbar ist.

In manchen erfindungsgemäßen Ausführungsformen ist die Verriegelungsvorrichtung eine Verriegelungsschraube oder ein Verriegelungsstift.

Im Folgenden werden die Begriffe Verriegelungsvorrichtung und Verriegelungsschraube synonym verwendet.

In gewissen erfindungsgemäßen Ausführungsformen ist die Aufnahmeeinrichtung eine Hülse.

Im Folgenden werden die Begriffe Aufnahmeeinrichtung und Hülse synonym verwendet.

Erfindungsgemäß ist der wenigstens eine Stützkörper wenigstens abschnittsweise formschlüssig mit dem länglichen Schaft und/ oder wenigstens abschnittsweise formschlüssig mit einem kassettenförmigen Einsatz lösbar verbunden. Der kassettenförmige Einsatz ist im länglichen Schaft positionierbar.

Erfindungsgemäß ist der formschlüssig mit dem länglichen Schaft oder der formschlüssig mit dem kassettenförmigen Einsatz verbundene Stützkörper zur Rotationssicherung (Verdrehsicherung) der in der Hülse angeordneten Verriegelungsschraube vorgesehen. Mittels der Rotationssicherung können Drehmomente, die von einer Arretiervorrichtung (z. B. eine Innensechskantschraube) zum Fixieren oder Verblocken der Hülse oder der Hülsen in der Kassette oder im Marknagel aufgebracht werden, vorteilhaft von der formschlüssigen Verbindung aufgenommen werden, ohne die Hülsen, und damit in den Hülsen angeordnete Verriegelungsschrauben, mitzudrehen.

Im Folgenden werden die Begriffe kassettenförmiger Einsatz und Kassette synonym verwendet.

In bestimmten erfindungsgemäßen Ausführungsformen ist die Kassette wenigstens abschnittsweise formschlüssig mit dem Verriegelungsmarknagel verbunden. Der Formschluss kann beispielsweise mittels eines Längsstegs an der Außenseite der Kassette und einer Längsnut an der Innenseite des Marknagels (Nut-Steg-Verbindung) erzielt werden. Alternativ oder zusätzlich kann an einem axialen Endbereich der Kassette, insbesondere an der stirnseitig geschlossenen Außenseite der Kassette, ein exzentrischer Absatz (exzentrisch in Bezug auf die Mittelachse der Kassette) angeordnet sein, der in eine Vertiefung im Marknagel formschlüssig einsetzbar ist.

In gewissen erfindungsgemäßen Ausführungsformen ist der Stützkörper mittels einer Nut-Steg-Verbindung mit dem länglichen Schaft lösbar verbunden. Insbesondere ist der Stützkörper scheibenförmig ausgebildet und umfasst am äußeren Umfang einen oder mehrere am Umfang angeordnete Absätze oder Stege. Die Stege können in Nuten im Schaft des Marknagels eingreifen und einen Formschluss erzeugen (formschlüssige Nut-Steg-Verbindung). Der Stützkörper kann anstatt einer Scheibenform gleichfalls eine andere Form aufweisen, beispielsweise eine Stabform (runder Querschnitt) oder eine Balkenform (eckiger Querschnitt). Der Stab oder der Balken kann formschlüssig in die Nut des Schafts des Marknagels eingreifen.

In manchen erfindungsgemäßen Ausführungsformen ist der Stützkörper mittels einer Nut-Steg-Verbindung mit der Kassette verbunden. Insbesondere ist der Stützkörper scheibenförmig ausgebildet und umfasst am äußeren Umfang einen oder mehrere am Umfang angeordnete Absätze oder Stege. Die Stege können in Nuten der Kassette eingreifen und einen Formschluss erzeugen (formschlüssige Nut-Steg-Verbindung). Der Stützkörper kann anstatt einer Scheibenform gleichfalls eine andere Form aufweisen, beispielsweise eine Stabform (runder Querschnitt) oder eine Balkenform (eckiger Querschnitt). Der Stab oder der Balken kann formschlüssig in die Nut der Kassette eingreifen.

In einigen erfindungsgemäßen Ausführungsformen weist der Schaft des Marknagels eine Hülse und einen Stützkörper mit einem Absatz am Umfang zur Rotationssicherung auf. Zur Montage kann zunächst die Hülse durch ein axial offenes Ende in den Schaft eingesetzt werden. Anschließend kann der Stützkörper in den Schaft eingesetzt werden. Der Stützkörper kann entweder ebenfalls durch das axial offene Ende in den Schaft eingesetzt werden oder durch eine Öffnung auf dem Umfang des Schafts (durch diese Öffnung wird später die Verriegelungsschraube hindurchgeführt), wobei die Öffnung eine Längsnut aufweist. Beispielsweise kann ein scheibenförmiger Stützkörper durch eine Drehung und ein Durchschieben längs einer Querachse des Stützkörpers in den Schaft eingeführt werden. Anschließend kann der Stützkörper in dem Schaft derart ausgerichtet werden, dass eine Senkrechte der scheibenförmigen Oberfläche des Stützkörpers in Achsrichtung des Schafts ausgerichtet wird. Weiterhin kann der Stützkörper um die Längsachse des Schafts derart gedreht werden, dass der Absatz des Stützkörpers in die Längsnut der Öffnung eingreift. Die Anordnung und die Dimensionen der Öffnung auf dem Umfang des Schafts, die Längsnut der Öffnung, die Positionen der Hülse und des Stützkörpers in dem Schaft (im montierten Zustand) werden insbesondere derart gewählt, dass der Stützkörper auf der Hülse aufliegt oder anliegt. Mittels dieser Anordnung kann später vorteilhaft die Verriegelungsschraube in der Hülse verblockt werden. Der Schaft kann mehrere Öffnungen auf dem Umfang aufweisen.

In einigen erfindungsgemäßen Ausführungsformen weist die Kassette eine Hülse und einen Stützkörper mit einem Absatz am Umfang zur Rotationssicherung auf. Zur Montage kann zunächst die Hülse durch ein axial offenes Ende in die Kassette eingesetzt werden. Anschließend kann der Stützkörper in die Kassette eingesetzt werden. Der Stützkörper kann entweder ebenfalls durch das axial offene Ende in die Kassette eingesetzt werden oder durch eine Öffnung auf dem Umfang der Kassette (durch diese Öffnung wird später die Verriegelungsschraube hindurchgeführt), wobei die Öffnung eine Längsnut aufweist. Beispielsweise kann ein scheibenförmiger Stützkörper durch eine Drehung und ein Durchschieben längs einer Querachse des Stützkörpers in die Kassette eingeführt werden. Anschließend kann der Stützkörper in der Kassette derart ausgerichtet werden, dass eine Senkrechte der scheibenförmigen Oberfläche des Stützkörpers in Achsrichtung der Kassette ausgerichtet wird. Weiterhin kann der Stützkörper um die Längsachse der Kassette derart gedreht werden, dass der Absatz des Stützkörpers in die Längsnut der Öffnung eingreift. Die Anordnung und die Dimensionen der Öffnung auf dem Umfang der Kassette, die Längsnut der Öffnung, die Positionen der Hülse und des Stützkörpers in der Kassette (im montierten Zustand) werden insbesondere derart gewählt, dass der Stützkörper auf der Hülse aufliegt oder anliegt. Mittels dieser Anordnung kann später vorteilhaft die Verriegelungsschraube in der Hülse verblockt werden. Die Kassette kann mehrere Öffnungen auf dem Umfang aufweisen.

In bestimmten erfindungsgemäßen Ausführungsformen weist der Schaft und/ oder die Kassette zwei oder mehrere Hülsen und zwei oder mehrere Stützkörper kaskadenförmig (reihenförmig) auf. Je nach Anzahl der Hülsen können mehrere Verriegelungsschrauben in die Hülsen eingebracht und der Marknagel im Knochen verschraubt werden.

In gewissen erfindungsgemäßen Ausführungsformen weist von zwei gegenüberliegenden Öffnungen auf dem Umfang des Schafts und/oder auf dem Umfang der Kassette nur eine Öffnung eine Nut auf, oder beide gegenüberliegenden Öffnungen. Bei mehreren Hülsen und Stützkörpern kann z. B. zunächst eine Hülse durch ein axial offenes Ende des Marknagels eingeführt werden (diese Hülse kann beispielsweise auf einem Absatz oder einem Zwischenboden in dem Marknagel positioniert werden). Anschließend kann ein Stützkörper von außen durch eine Öffnung (zur Aufnahme der Verriegelungsschraube) eingelegt und rotationsfixiert in der Längsnut mittels des Stegs angeordnet werden. Im nächsten Schritt kann eine weitere Hülse durch das axial offene Ende eingeführt und ein weiterer Stützkörper durch eine weitere Öffnung eingebracht und ausgerichtet werden. Auf diese Art und Weise können mehrere Hülsen und Stützkörper in dem Marknagel und/oder in der Kassette angeordnet werden.

In manchen erfindungsgemäßen Ausführungsformen wird zunächst die Kassette mit den Stützkörpern und den Hülsen montiert und anschließend in den Schaft des Marknagels eingeführt.

In einigen erfindungsgemäßen Ausführungsformen ist die Kassette zum Einsetzen von wenigstens zwei Hülsen und zwei scheibenförmigen Stützkörpern ausgestaltet, wobei die Kassette wenigstens eine Längsnut aufweist. Wenigstens eine Längsnut kann sich von dem axial offenen Ende der Kassette bis mindestens über die halbe axiale Länge der Kassette erstrecken. Insbesondere ist die Länge der Längsnut derart ausgelegt, dass zunächst eine Hülse durch das axial offene Ende der Kassette einlegbar ist und anschließend ein Stützkörper, der nachfolgend in die Kassette eingelegt wird, auf dieser Hülse aufliegt oder anliegt. Der Stützkörper wird mittels eines Absatzes auf seinem Umfang in der Nut rotationssicher positioniert. Nachfolgend können eine wenigstens zweite Hülse und ein wenigstens zweiter Stützkörper in die Kassette eingelegt werden. Der zweite Stützkörper kann ebenfalls mittels eines Absatzes am Umfang des Stützkörpers in die sich über die wenigstens halbe axiale Länge der Kassette erstreckende Längsnut eingelegt und rotationssicher positioniert werden. Alternativ kann der wenigstens zweite Stützkörper mit einem Absatz in eine weitere Längsnut in der Kassette eingelegt werden.

In bestimmten erfindungsgemäßen Ausführungsformen weist die Kassette keine Längsnut auf. Beispielsweise kann die Kassette zum Einlegen einer ersten Hülse, eines Stützkörpers (ohne Absatz am Umfang) und einer zweiten Hülse (durch eine axiale Öffnung) ausgestaltet sein, wobei die erste Hülse, der Stützkörper und die zweite Hülse in der Kassette aneinander anliegen, beispielsweise mittels zweier konkaver, muldenförmiger Vertiefungen auf zwei gegenüberliegenden Seiten eines scheibenförmigen Stützkörpers. Eine derart ausgestaltete Kassette kann in den Schaft des Marknagels eingeführt werden. Zur Rotationssicherung der Hülsen in der Kassette, insbesondere zur Rotationssicherung von Verriegelungsschrauben, die in die Hülsen eingeführt werden können, kann ein weiterer Stützkörper, nach Einbringung der Kassette in den Schaft, in den Schaft eingeführt werden. Dieser, beispielsweise scheibenförmige, weitere Stützkörper kann einen Absatz auf dem Umfang aufweisen, um mittels dieses Absatzes in eine Längsnut in dem Schaft eingeführt zu werden. Diese Längsnut kann an einem axial offenen Ende des Schafts, beispielsweise in einem Innengewinde, angeordnet sein. Dieser weitere Stützköper liegt insbesondere an der zweiten Hülse der Kassette an. Die gesamte Anordnung von Kassette und weiterem Stützkörper kann beispielsweise mittels einer Arretiervorrichtung, z. B. einer Schraube, verblockt werden. Bei diesem Verblocken kann die Rotationssicherung des weiteren Stützkörpers vorteilhaft sein, wenn eine Schraube als Arretiervorrichtung in ein Innengewinde am axialen Schaftende eingeschraubt wird.

In gewissen erfindungsgemäßen Ausführungsformen umfasst ein Gewindeabschnitt, insbesondere ein Innengewindeabschnitt, an einem axialen Ende des Marknagels eine Nut, insbesondere eine Längsnut (in Bezug auf die Längsachse des Marknagels), zur Aufnahme des Stützkörpers.

In manchen erfindungsgemäßen Ausführungsformen ist der Stützkörper scheibenförmig ausgestaltet und weist in einem zentralen Bereich eine konvexe oder eine konkave Profilierung auf. Die konkave Profilierung kann muldenförmig sein, wobei die Oberfläche der Mulde eine mikrostrukturiert oder makrostrukturiert aufgeraute Oberfläche aufweisen kann. Derartige Strukturen können mittels Sandstrahlens hergestellt werden. Weiterhin kann die Muldenstruktur eine Rändelform aufweisen.

In einigen erfindungsgemäßen Ausführungsformen kann sich die konvexe Oberflächenstruktur des Stützkörpers unter Aufbringung eines mechanischen Drucks (beispielsweise mittels einer schraubenförmigen Arretiervorrichtung) in eine anliegende Hülse als Aufnahmevorrichtung einpressen und plastisch oder elastisch verformen.

In bestimmten erfindungsgemäßen Ausführungsformen ist der Stützkörper ringförmig ausgestaltet und weist in einem zentralen Bereich des Stützkörpers ein offenes Profil auf. Dieses offene Profil kann konturiert sein. Der ringförmige Stützkörper kann einen radialen Schlitz aufweisen. Unter Aufbringung eines mechanischen Drucks auf den geschlitzten Ring kann sich der Außendurchmesser des Rings erweitern. Wenn beispielsweise der geschlitzte Ring in den Schaft des Marknagels oder in die Kassette eingeführt wird, kann sich der geschlitzte Ring unter Aufbringung eines mechanischen Drucks nach außen an die Innenwand des Schafts oder der Kassette pressen. Damit kann ein Kraftschluss erfolgen und der Ring im Marknagel oder in der Kassette lagestabil verklemmt werden.

In gewissen erfindungsgemäßen Ausführungsformen weist der Stützkörper eine stabförmige Kontur auf. Die stabförmige Kontur kann rund oder eckig (z. B. rechteckig) sein. Der Stützkörper kann aus Metall, Kunststoff oder einem anderen Material hergestellt sein. Der Stützkörper kann unter Aufbringung eines mechanischen Drucks elastisch oder plastisch verformt werden.

In manchen erfindungsgemäßen Ausführungsformen ist die Hülse aus einem ersten und einem zweiten Material gefertigt oder weist diese Materialien auf. Die Hülse kann ein Material zur Selbstsicherung einer Verriegelungsschraube aufweisen. Dieses Material kann in Form eines Inlays, z. B. aus Kunststoff, zum Verhindern eines ungewollten Herausschraubens der Verriegelungsschraube, mit der Hülse verbunden sein.

In einigen erfindungsgemäßen Ausführungsformen ist die Hülse ganz oder teilweise beschichtet. Eine Beschichtung kann beispielsweise Kunststoff oder gesinterte Keramik aufweisen.

In manchen erfindungsgemäßen Ausführungsformen ist die Oberfläche der Hülse oberflächenbehandelt. Beispielsweise kann die Oberfläche mikrostrukturiert oder makrostrukturiert sein. Oberflächenbehandlungen können mittels Sandstrahlens oder mittels abrasiver Verfahren, z. B. Schleifverfahren, durchgeführt oder bearbeitet werden.

In bestimmten erfindungsgemäßen Ausführungsformen weist die Hülse wenigstens einen Gewindegang zur Aufnahme der Verriegelungsschraube auf.

In gewissen erfindungsgemäßen Ausführungsformen weist die Öffnung (oder mehrere Öffnungen) im Schaft des Marknagels oder in der Kassette zur Aufnahme der Verriegelungsschraube wenigstens eine Senkung zum Einsetzen der Verriegelungsschraube auf. Mittels der Senkung kann ein Formschluss des Verriegelungsschraubenkopfes oder der Verriegelungsschraube mit dem Marknagel oder der Kassette erreicht werden.

In einigen erfindungsgemäßen Ausführungsformen weist wenigstens eine Öffnung im Schaft des Marknagels oder in der Kassette im Bereich ihres äußeren Querschnitts wenigstens eine Abschrägung auf. Die Abschrägung kann als Senkung oder Fase bezeichnet werden. Die Abschrägung kann einen Winkel - in Bezug auf eine Achse senkrecht zur Mittelachse des Marknagels von beispielsweise 45 Grad, 60 Grad oder 30 Grad aufweisen. Mittels der Abschrägung kann eine Verriegelungsschraube in einer Hülse, welche in dem Marknagel im Bereich der Öffnung angeordnet ist, in einem größeren Winkelbereich um die Achse senkrecht zur Mittelachse des Marknagels bewegt werden als bei einer Öffnung ohne Abschrägung. Mittels des größeren Winkelbereichs kann die Verriegelungsschraube in dem Marknagel und im Knochen vorteilhaft in einem größeren Verstellbereich eingebracht werden.

In manchen erfindungsgemäßen Ausführungsformen weist der Marknagel eine Arretiervorrichtung zum Verblocken der wenigstens einen Hülse auf. Die Arretiervorrichtung kann eine Schraube sein, die in ein Innengewinde an einem axialen Ende des Marknagels eingeschraubt wird. Insbesondere ist die Schraube ein Gewindestift.

In einigen erfindungsgemäßen Ausführungsformen weist der Marknagel eine Exzentereinrichtung zum Verblocken der wenigstens einen Hülse auf. Die Exzentereinrichtung kann eine elastische Gegenkraft nach Aufbringen einer Kraft zum Verblocken der Hülse mittels einer Arretiervorrichtung erzeugen. Mittels der Exzentereinrichtung können Druck- und/oder Zugkräfte auf die Hülse ausgeübt werden und die Hülse somit arretieren oder lagestabil fixieren.

Das offenbarte, nicht-erfindungsgemäße Verfahren zum Verblocken von wenigstens einer Hülse eines Marknagels weist den Schritt des Verpressens der wenigstens einen Hülse und wenigstens eines Stützkörpers mittels eines Presskörpers auf. Der Presskörper ist an einem axialen Endbereich des Marknagels angeordnet. Die Hülse kann mittels des Presskörpers elastisch deformiert werden und somit die Verriegelungsschraube lagestabil verklemmen oder einklemmen.

In einigen erfindungsgemäßen, beispielhaften Ausführungsformen weist der Verriegelungsmarknagel einen inneren Hohlraum auf.

In bestimmten erfindungsgemäßen, beispielhaften Ausführungsformen weist der erfindungsgemäße Verriegelungsmarknagel, etwa an einem distalen Ende seines inneren Hohlraums, wenigstens eine Vertiefung auf. Die Vertiefung kann als Mulde oder Aussparung bezeichnet werden. Die Oberfläche der Vertiefung kann eine gleiche oder ähnliche Oberflächenform und/oder Oberflächenbeschaffenheit aufweisen wie die Stützkörper. Die Vertiefung kann funktional in der gleichen Weise wirken die Stützkörper, also beispielsweise als erhöhter Reibwiderstand gegen eine unbeabsichtigte Verdrehung oder Rotation der Hülsen.

In manchen erfindungsgemäßen, beispielhaften Ausführungsformen weist der erfindungsgemäße Verriegelungsmarknagel ein axiales Loch auf, und am Lochgrund/Lochabsatz befindet sich die vorstehend beschriebene Vertiefung. Dabei ist der Lochgrund nicht zwingend als geschlossen zu betrachten, sondern kann auch als Absatz bezeichnet werden und ein weiteres axiales Loch kann sich daran anschliessen.

In einigen erfindungsgemäßen, beispielhaften Ausführungsformen weist der erfindungsgemäße Verriegelungsmarknagel einen Inlay-Ring auf. Der Inlay-Ring kann am distalen Ende des inneren Hohlraums des Verriegelungsmarknagels, insbesondere zwischen einer Hülse und der oben beschriebenen, optional vorgesehenen, Vertiefung, angeordnet sein. Mittels einer axialen Kraft auf einen am proximalen, offenen Ende des Verriegelungsmarknagels angeordneten Stützkörper kann die distale Hülse auf oder gegen den Inlay-Ring gedrückt werden. Mittels dieser axialen Kraft kann eine Vorspannung auf die distale oder proximale Hülse, und gegebenenfalls auf weitere Hülsen im Hohlraum des Verriegelungsmarknagels, vorteilhaft ausgeübt werden, so dass eine rotationsstabile und/oder lagestabile Positionierung, insbesondere nach einer vorausgegangenen Ausrichtung der Hülse(n), erzielt werden kann.

In gewissen erfindungsgemäßen, beispielhaften Ausführungsformen weist die entsprechende Öffnung am Nagelkörper aufgrund der Verformung des Inlays eine entsprechende Form auf, die beispielsweise langlochartig ist.

In manchen erfindungsgemäßen, beispielhaften Ausführungsformen ist der Inlay-Ring aus einem elastisch verformbaren Material hergestellt oder weist ein solches auf. Das Material des Inlay-Rings kann ein Kunststoff sein, beispielsweise Polyetheretherketon (abgekürzt PEEK).

In gewissen erfindungsgemäßen, beispielhaften Ausführungsformen weist der erfindungsgemäße Verriegelungsmarknagel einen Inlay-Stab auf. Der Inlay-Stab ist insbesondere am distalen Ende des inneren Hohlraums des Verriegelungsmarknagels im Bereich der Vertiefung oder in der Vertiefung angeordnet. Der Inlay-Stab kann in der gleichen oder in einer ähnlichen Weise wirken und verwendet werden wie der vorstehend beschriebene Inlay-Ring. Der Inlay-Stab kann aus demselben oder einem ähnlichen Material wie der Inlay-Ring hergestellt sein oder dieses aufweisen. Die Vorspannung kann analog zu der Vorspannung auf den Inlay-Ring aufgebaut werden und/oder wirken. Bei einer Erhöhung der axialen Kraft über die Vorspannung hinaus können der proximal angeordnete Stützkörper und die distal angeordnete Hülse, und gegebenenfalls weitere Stützkörper und Hülsen innerhalb des Hohlraums des Verriegelungsmarknagels, weiter axial verschoben und das Inlay (etwa der Inlay-Stab oder der Inlay-Ring), insbesondere hierdurch, soweit verformt oder gestaucht werden, bis der Stützkörper in die Vertiefung hineingedrückt wird und ein Formschluss zwischen den Oberflächen der distalen Hülse und der Vertiefung entsteht oder gebildet wird. Dieser Formschluss kann als Verblockung des Stützkörpers in dem Verriegelungsmarknagel bezeichnet werden.

In bestimmten erfindungsgemäßen, beispielhaften Ausführungsformen weist der erfindungsgemäße Verriegelungsmarknagel zylinderförmige Inlays (eines oder mehrere) auf, oder es sind zylinderförmige Inlays in dem erfindungsgemäßen Verriegelungsmarknagel angeordnet. Die zylinderförmigen Inlays können radial in Bohrungen oder Öffnungen in dem Schaft des Verriegelungsmarknagels angeordnet sein. Die zylinderförmigen Inlays können in den inneren Hohlraum des Verriegelungsmarknagels hineinragen. Die zylinderförmigen Inlays können vorteilhaft ein Durchrutschen oder Verschieben der im Hohlraum des Verriegelungsmarknagels angeordneten distalen Hülse in die (distale) Vertiefung hinein verhindern. Die zylinderförmigen Inlays können analog dem zuvor beschriebenen Inlay-Ring und/oder dem Inlay-Stab wirken.

Demzufolge wird in einigen beispielhaften erfindungsgemäßen Ausführungsformen ein Marknagel zum Einbringen in einen Röhrenknochen vorgeschlagen, der einen Schaft mit einer Längsachse aufweist. Der Schaft weist wenigstens eine Durchgangsöffnung zum Einbringen wenigstens einer Verriegelungseinrichtung, insbesondere einer Verriegelungsschraube oder eines Verriegelungsstifts, in ein Inneres des Marknagels und/oder zum Hindurchführen durch den Marknagel auf.

Der Marknagel weist in einigen beispielhaften erfindungsgemäßen Ausführungsformen wenigstens eine Aufnahmeeinrichtung zum Aufnehmen wenigstens eines Abschnitts der Verriegelungseinrichtung auf.

Die wenigstens eine Aufnahmeeinrichtung ist in einigen beispielhaften erfindungsgemäßen Ausführungsformen in wenigstens einer ihrer Raumachsen in Bezug zur Längsachse des Schafts bewegbar oder ausrichtbar. Sie ist insbesondere drehbar oder verschwenkbar, am Marknagel, innerhalb des Schafts und/oder in der Durchgangsöffnung angeordnet.

Der Marknagel weist in einigen beispielhaften erfindungsgemäßen Ausführungsformen wenigstens eine Einrichtung, insbesondere eine Arretiereinrichtung und/oder eine Ausgestaltung von Verriegelungseinrichtung und/oder Aufnahmeeinrichtung, zum Unterbinden der Ausrichtbarkeit der wenigstens einen Aufnahmeeinrichtung in Bezug zur Längsachse und/oder zu einer hierzu senkrechten Querachse auf.

Die wenigstens eine Aufnahmeeinrichtung weist in einigen beispielhaften erfindungsgemäßen Ausführungsformen wenigstens eine durch sie hindurchreichende Führungsdrahtöffnung zum Aufnehmen, Hindurchführen oder Umgeben eines Führungsdrahts auf. Die wenigstens eine Führungsdrahtöffnung kann synonym auch als Führungsdrahtdurchlass oder als Führungsdrahtführung bezeichnet werden.

Unter einem Führungsdraht wird in gewissen erfindungsgemäßen Ausführungsformen ein Draht (oder eine hierfür ebenfalls einsetzbare und/oder in der Praxis bereits eingesetzte Struktur) verstanden, welcher vor dem Einbringen eines orthopädischen oder chirurgischen Instruments und/oder eines Implantats in das Gewebe, beispielsweise in den Knochen, eingebracht wird. Der Führungsdraht ist dabei vorgesehen, um an ihm entlang, ihn umgebend, ihn einschließend, mit ihm verbunden und/oder durch ihn geführt in einem späteren, zweiten Arbeitsschritt ein Instrument oder ein Implantat in jenes Gewebe einzubringen, in welches der Führungsdraht in einem früheren, ersten Arbeitsschritt bereits eingebracht wurde. Der Führungsdraht dient in diesen Ausführungsformen somit als *path finder,* Wegbereiter oder Leitstruktur für das Instrument oder das Implantat, indem er dessen ortsgerechte Einbringung in dasselbe Gewebe vorbereitet und/oder erleichtert.

So ist die wenigstens eine Führungsdrahtöffnung des erfindungsgemäßen Marknagels in manchen beispielhaften Ausführungsformen verschieden von einer Durchgangsöffnung der Aufnahmeeinrichtung, durch welche die Verriegelungseinrichtung durch die Aufnahmeeinrichtung hindurchtritt.

In gewissen erfindungsgemäßen Ausführungsformen ist die Führungsdrahtöffnung zu klein im Durchmesser, um eine Verriegelungsschraube durch sie hindurch zu führen.

In manchen erfindungsgemäßen Ausführungsformen ist der Marknagel ein Arthrodesenagel, beispielsweise zum Versteifen von Gelenken.

In einigen beispielhaften Ausführungsformen des erfindungsgemäßen Marknagels verläuft die wenigstens eine Führungsdrahtöffnung in manchen ihrer Abschnitte durch die Durchgangsöffnung der Aufnahmeeinrichtung hindurch, in anderen Abschnitten hingegen nicht.

In bestimmten beispielhaften Ausführungsformen des erfindungsgemäßen Marknagels weist die wenigstens eine Führungsdrahtöffnung einen Durchmesser oder eine Breite in einem Bereich zwischen 1 bis 5 mm, vorzugsweise zwischen 2 und 4 mm, vorzugsweise 3 mm auf. In gewissen beispielhaften erfindungsgemäßen Ausführungsformen weist die Führungsdrahtöffnung einen Durchmesser oder eine Breite von maximal 3 mm auf. Dies erlaubt vorteilhaft eine ausreichende Dicke und Festigkeit der die Führungsdrahtöffnung umgebenden Strukturen.

In einigen beispielhaften Ausführungsformen des erfindungsgemäßen Marknagels verläuft die wenigstens eine Führungsdrahtöffnung nicht nur durch die eine oder die mehreren Aufnahmeeinrichtung(en), sondern kann ferner durch optional vorgesehene Zwischenkörper, welche zwischen benachbarten Aufnahmeeinrichtungen optional vorgesehen sein können, verlaufen. Entsprechende Öffnungen oder Durchlässe können auch hierin vorgesehen sein.

Das Vorsehen der Führungsdrahtöffnung erlaubt es in einigen beispielhaften erfindungsgemäßen Ausführungsformen vorteilhaft, den erfindungsgemäßen Marknagel entlang eines durch den Marknagel verlaufenden Führungsdrahts einzubringen. Der Marknagel wird dabei erst dann in den Knochen eingetrieben, wenn seine künftige Lage und sein Einbringweg anhand des zuvor eingebrachten Führungsdrahts sondiert und festgelegt sowie ggf. auch - beispielsweise röntgentechnisch - überprüft sind. Auf diese Weise darf der Chirurg vor dem Einbringen des Marknagels mit größerer Sicherheit davon ausgehen, dass der Marknagel so zum Liegen kommen wird, wie gewünscht.

Ein weiterer Vorteil von Führungsdrahtöffnungen kann in einigen beispielhaften erfindungsgemäßen Ausführungsformen darin bestehen, dass mittels eines hierin eingeführten Führungsdrahts die Stellung der Aufnahmeeinrichtung während des mehr oder weniger gewaltsamen Einschlagens des Marknagels in den Knochen zuverlässig gewahrt wird. Der Führungsdraht verhindert ein unbeabsichtigtes Verdrehen der einen oder der mehreren Aufnahmeeinrichtungen, welche sich durch Erschütterungen während des Einbringens usw. ergeben können. Aufgrund der mittels des Führungsdrahts gewahrten Stellung der Aufnahmeeinrichtung bezogen auf den Marknagel oder innerhalb des Marknagel kann in einigen beispielhaften erfindungsgemäßen Ausführungsformen sichergestellt werden, dass Öffnungen der einzelnen Aufnahmeeinrichtungen, durch welche nach Einbringen des Marknagels der oder die Verriegelungsstift(e) gesteckt werden, auch nach dem Einbringen des Marknagels in den Knochen mit jenen Öffnungen des Marknagels, durch welche die Verriegelungsstifte ebenfalls gesteckt werden müssen, noch fluchten (oder gar erstmals fluchten). Dies erleichtert in einigen beispielhaften erfindungsgemäßen Ausführungsformen das Einbringen der Verriegelungsschrauben in Knochen und Marknagel merklich.

In einigen beispielhaften Ausführungsformen des erfindungsgemäßen Marknagels weist dieser auch jenseits der Aufnahmeeinrichtung(en) wenigstens eine Öffnung oder einen Führungsdrahtdurchlass für einen Führungsdraht auf, welche den Führungsdraht innerhalb des Marknagels zumindest im Bereich der Öffnung/des Durchlasses führt. Der Führungsdraht kann hierzu vergleichsweise eng durch die Öffnung geführt werden, oder diese kann nur geringfügig weiter als der Durchmesser des Führungsdrahts sein. Dies erlaubt ein wirkungsvolles Führen des Führungsdrahts im Bereich der Öffnung mittels des Führungsdrahtdurchlasses und relativ zum Marknagel.

In gewissen beispielhaften Ausführungsformen des erfindungsgemäßen Marknagels ist/sind die Führungsdrahtöffnung(en) der Aufnahmeeinrichtung(en) nicht in einem Symmetriemittelpunkt der Aufnahmeeinrichtung(en) vorgesehen. Auf diese Weise ist eine wie oben beschriebene Ausrichtung der Aufnahmeeinrichtung(en) selbst dann vorteilhaft möglich, wenn die Aufnahmeeinrichtung beispielsweise eine Kugel ist.

In einigen beispielhaften Ausführungsformen des erfindungsgemäßen Marknagels sind die Durchgangsöffnungen für die Verriegelungsschraube(n) Durchbrüche, in deren Bereich die Mantelfläche des Marknagels durchbrochen oder gegenüber einem Äußeren des Marknagels geöffnet ist.

In manchen beispielhaften Ausführungsformen des erfindungsgemäßen Marknagels ist die wenigstens eine Aufnahmeeinrichtung kein integraler Bestandteil des Marknagels sondern ein hiervon separates, ggf. auswechselbares Bauteil.

In einigen beispielhaften Ausführungsformen des erfindungsgemäßen Marknagels ist die Aufnahmeeinrichtung im Bereich zumindest einer Öffnung des Marknagels als zumindest in einer Raumrichtung - bspw. in einer Richtung senkrecht zu der Längsachse des Knochen- oder Marknagelschaftes- und/ oder in einer Ebene, insbesondere einer die zuvor genannte Richtung enthaltende Ebene - ausrichtbar, oder bewegbar oder verschiebbar oder verschwenkbar in dem Schaft und/ oder am Schaft angeordnet und/ oder gelagert.

Die Aufnahmeeinrichtung weist in einigen beispielhaften Ausführungsformen des erfindungsgemäßen Marknagels eine Öffnung oder Bohrung zur Aufnahme einer Verriegelungsschraube oder mehrere solcher oder anderer Verriegelungseinrichtungen auf. Nach Einsetzen der Verriegelungsschraube(n) in den Schaft kann diese Aufnahmeeinrichtung wiederum verriegelt bzw. blockiert bzw. arretiert werden.

In manchen beispielhaften Ausführungsformen des erfindungsgemäßen Marknagels sind eine, mehrere oder alle der vorhandenen Aufnahmeeinrichtungen als Hülsen ausgestaltet oder weisen wenigstens eine solche auf.

Es gilt hierin, dass wenn im Folgenden daher von einer Hülse die Rede ist, dieser Begriff nicht hierauf beschränkend zu verstehen ist. Das für die Hülse jeweils Gesagte trifft in anderen beispielhaften erfindungsgemäßen Ausführungsformen auch auf die Aufnahmeeinrichtung im weitesten Sinne zu, sofern der Fachmann hierin keinen Widerspruch erkennt.

In einigen beispielhaften Ausführungsformen des erfindungsgemäßen Marknagels kann die Verschwenkbarkeit der Hülse und ihre Nicht-Verschwenkbarkeit nach Verriegelung oder Blockung an einer zugelassenen oder unterbundenen Bewegung, insbesondere Neigbarkeit oder Drehbarkeit, einer in Bezug auf die Hülse festlegbaren virtuellen Achse oder Geraden bestimmt oder festgemacht werden. Diese Achse kann eine Längsachse oder eine Symmetrieachse der Öffnung oder Bohrung der Hülse sein.

In manchen beispielhaften Ausführungsformen des erfindungsgemäßen Marknagels kann die Aufnahmeeinrichtung eine Einrichtung mit wenigstens einer vorgefertigten Durchgangsöffnung für die Verriegelungsschraube sein. Unter den Begriff "Hülse" oder Aufnahmeeinrichtung fällt neben der vom Fachmann unter Hülse oder Aufnahmeeinrichtung verstandenen Struktur erfindungsgemäß auch jede Einrichtung, welche keine vorgefertigte Durchgangsöffnung aufweist, sondern bei welcher ein Durchlass für die Verriegelungsschraube beim Einbringen der Verriegelungsschraube und ggf. auch erst durch das Einbringen entsteht.

In einigen beispielhaften Ausführungsformen des erfindungsgemäßen Marknagels umfasst "Hülse" oder Aufnahmeeinrichtung somit auch eine Masse, insbesondere eine aushärtbare Masse wie Knochenzement, welche zu einem frühen ersten Zeitpunkt ein Ausrichten des oder der durch sie hindurch geführte(n) Verriegelungsschraube(n) erlaubt, nicht mehr aber zu einem späteren zweiten Zeitpunkt. Auch eine Arretierung mittels eines Materials, welches bei unterschiedlichen Umgebungstemperaturen unterschiedliche Formen einnimmt und sich bei Wärme ausdehnen oder zusammenziehen kann, wird, wenn der Arretiereffekt durch diese Materialeigenschaft bewirkt oder begünstigt wird, in manchen beispielhaften Ausführungsformen erfindungsgemäß unter der Aufnahmeeinrichtung oder Hülse verstanden.

Die Hülse ist in manchen beispielhaften Ausführungsformen des erfindungsgemäßen Marknagels eine Walze mit einer Walzenachse, welche bspw. senkrecht zu der Längsachse des Schafts des Verriegelungsnagels steht. In anderen beispielhaften Ausführungsformen des erfindungsgemäßen Marknagels ist sie vorteilhaft kugelförmig oder von elliptischem oder elliptoidem Durchmesser, so dass sie in mehreren, vorzugsweise in allen Raumachsen frei schwenkbar im Schaft gelagert ist.

Die Aufnahmeeinrichtung ist in einigen beispielhaften Ausführungsformen des erfindungsgemäßen Marknagels aus mehreren Teilen und/ oder mehreren Materialien zusammengesetzt (Composit). Beispielsweise kann ein Kunststoffring als Teil der Aufnahmeeinrichtung zum Einsatz kommen. Er kann ein ungewolltes Herausdrehen der Verriegelungsschraube(n) vorteilhaft verhindern.

In einigen beispielhaften Ausführungsformen des erfindungsgemäßen Marknagels weist dieser wenigstens oder genau eine, insbesondere zum Schaft des Marknagels koaxial und/oder im Wesentlichen parallel zur Längsachse des Marknagels angeordnete, Klemmeinrichtung, beispielsweise eine Klemmschraube, zum Unterbinden der Ausrichtbarkeit der wenigstens einen oder von mehreren oder allen Aufnahmeeinrichtungen und/oder zur Fixierung von deren Lage auf.

Unter der "Bohrung", welche in der Aufnahmeeinrichtung vorgesehen ist, wird erfindungsgemäß eine Bohrung verstanden, wie sie dem Fachmann bekannt ist. Zusätzlich kann erfindungsgemäß auch eine Durchgangsöffnung zur Aufnahme der Verriegelungsschraube oder eines Abschnitts hiervon vorgesehen sein, welche der Fachmann nicht als eine Bohrung ansieht. Eine solche Durchgangsöffnung kann bspw. gegossen oder bei einem Gießvorgang (mit-)entstanden sein. Beim Gießen muss - anders als beim Erzeugen einer Bohrung kein Hülsenmaterial abgetragen oder beseitigt werden. Eine solche nicht gebohrte Durchgangsöffnung kann ferner durch Zusammenbringen mehrerer Teil-Aufnahmeeinrichtungen mit entsprechenden Aussparungen zustande kommen.

Unter einer Verriegelung wird in manchen beispielhaften Ausführungsformen des erfindungsgemäßen Marknagels ein Verhindern einer Bewegung - z. B. des Marknagels - in einer Längsachse hiervon oder um seine Längsachse herum nach Abschluss dessen Einbringens in den Knochen verstanden. Es wird im Zusammenhang mit der vorliegenden Erfindung jedoch auch ein Fixieren von Frakturfragmenten oder anderen Knochenabschnitten verstanden. Unter Fixieren wird auch der Versuch verstanden, den Abstand zwischen Knochenabschnitten oder -fragmenten zu bewahren. Ein Sintern, insbesondere ein ärztlich gewolltes Sintern, der Knochenabschnitte, bei welchem sich der Abstand trotz Verriegelung in Begrenztem Umfang verändern darf, ist hierbei dem Fixieren nicht abträglich. Daher kann eine Verriegelungsschraube oder -einrichtung im Sinne der vorliegenden Erfindung sowohl eine Einrichtung zum Fixieren des Marknagels im Knochen, als auch eine Einrichtung zum Sichern von wenigstens zwei Knochen oder -abschnitten oder -fragmenten relativ zueinander sein. Mit einer Konstruktion eines Marknagels gemäß der Erfindung können beim Einsetzen einer Verriegelungsschraube in den Marknagel in manchen beispielhaften Ausführungsformen geringe Fehlstellungen der Verriegelungsschraube quasi automatisch ausgeglichen werden. Zudem hat der Operateur, insbesondere bei einer kugelförmigen Hülse, die in drei Freiheitsgraden ausrichtbar ist, die Möglichkeit, noch während der Operation den Winkel der den Marknagel durchdringenden Verriegelungsschraube(n) bspw. zum Schaft und/oder die Ausrichtung der Verriegelungsschraube(n) im Raum variieren zu können. Hierdurch hat der Operateur eine Möglichkeit, die Frakturfragmente des Knochens oder des Gelenkes präzise zu repositionieren beziehungsweise anatomisch korrekt zu adaptieren, bzw. zu komprimieren.

Die Verriegelung der Aufnahmeeinrichtung mit der Verriegelungsschraube, beziehungsweise die Lagefixierung oder Blockierung der Aufnahmeeinrichtung, kann auf mehrere Arten erfolgen. Die Aufnahmeeinrichtung, die nur bevorzugt aus Kunststoff ist, und die unabhängig hiervon ähnlich wie bekannte Dübel mit einem Schlitz versehen sein kann, kann beispielsweise durch eine konische oder anders geeignete Ausbildung der Verriegelungsschraube aufgeweitet und fixiert werden. Ebenso ist es möglich, diese Lagefixierung der Aufnahmeeinrichtung mit der Verriegelungsschraube durch eine zum Schaft des Marknagels bspw. koaxial und/oder im Wesentlichen parallel angeordnete Klemmschraube zu erreichen. Eine solche Klemmschraube kann dabei auch bspw. als ein Passstift oder eine verriegelbare Einrichtung oder jede andere Einrichtung ausgestaltet sein bzw. durch diese ersetzt sein, welche eine Druck- oder Zugwirkung auf die Aufnahmeeinrichtung ausüben und zu deren Immobilisierung im Raum oder in wenigstens einer Ebene führen kann. Falls mehrere Aufnahmeeinrichtungen im Marknagel vorgesehen sind, können diese fixiert, insbesondere positions-, und/oder winkel- und/oder lagefixiert, werden, indem bspw. zwischen den einzelnen Aufnahmeeinrichtungen Zwischenkörper vorgesehen sind, wobei dann die einzelnen Aufnahmeeinrichtungen durch eine Einrichtung, z. B. durch eine Klemmschraube, fixiert werden. Auch Kombinationen der zuvor genannten und weiterer, dem Fachmann bekannten Einrichtungen der oben genannten Art zum Erzielen einer Arretierung oder Fixierung sind von der Erfindung umfasst.

Eine solche Einrichtung - wie Zwischenkörper - ist jedoch nicht unentbehrlich. Vielmehr können die Aufnahmeeinrichtungen in einigen beispielhaften erfindungsgemäßen Ausführungsformen alternativ auch gegeneinander verblockt werden bei direktem Kontakt der Aufnahmeeinrichtungen miteinander.

Die Zwischenkörper können in einigen beispielhaften erfindungsgemäßen Ausführungsformen massiv oder mit wenigstens einem Hohlraum ausgestaltet sein. Durch die Hohlräume kann sich eine größere Deformität der Zwischenkörper und/oder eine Erhöhung der Reibung zur Aufnahmeeinrichtung ergeben, was neben der Reibung auch die Winkelstabilität erhöht.

Die Zwischenkörper können in einigen beispielhaften erfindungsgemäßen Ausführungsformen so ausgeführt sein, dass sie ähnlich einer Verzahnung eine formschlüssige Verbindung mit der Aufnahmeeinrichtung eingehen können, um so bei der Verblockung einen erhöhten Reibwiderstand leisten zu können und damit die Winkelstabilität zu erhöhen.

Der erfindungsgemäße Marknagel kann in einigen beispielhaften erfindungsgemäßen Ausführungsformen in einem modularen Aufbau derart ausgestaltet sein, dass der Marknagel eine koaxiale Bohrung oder Öffnung aufweist, in welche ein Einsatz mit wenigstens einer Aufnahmeeinrichtung oder eine zusammenhängende Einheit von Aufnahmeeinrichtungen und Zwischenkörpern eingebracht werden kann. Diese Ausgestaltung erlaubt vorteilhaft, dass vor dem Einbringen des Einsatzes die Öffnung für eine Kanülierung nutzbar ist mit den für den Fachmann im Zusammenhang mit der Kanülierung bekannten Vorteilen.

Aufnahmeeinrichtungen können dabei in einigen beispielhaften erfindungsgemäßen Ausführungsformen anstelle mit Verriegelungsschrauben auch mit Platzhaltern besetzt werden. Dies hat den Vorteil, dass eine nicht mit einer Verriegelungsschraube belegte Aufnahmeeinrichtung mittels des Platzhalters einen gewünschten Kraftfluss über sie hinweg erlaubt, wenn nach Abschluss des Einbringens des Marknagels in den Knochen die axiale Verspannung für eine winkelstabile Sicherung genutzt wird.

Es wird darauf hingewiesen, dass der erfindungsgemäße Marknagel zusammen mit einer oder mehreren Verriegelungsschrauben verwendet werden kann. Daher ist die Verwendung der Begriffe "Verriegelungsschraube" und "Verriegelungsschrauben" in der vorliegenden Beschreibung austauschbar: Wo von "Verriegelungsschraube" die Rede ist, sind auch "Verriegelungsschrauben" zu verstehen und umgekehrt, wo immer dies technisch möglich ist. Dabei steht der Begriff "Verriegelungsschraube" für eine Verriegelungseinrichtung allgemein.

Daher kann die Verriegelungsschraube in einigen beispielhaften erfindungsgemäßen Ausführungsformen ferner als Verriegelungsstift ausgestaltet sein. Ein Gewinde ist erfindungsgemäß nicht erforderlich. Das Vorsehen einer Passung oder sonstigen Einrichtung zum Erhöhen der Reibung zwischen Aufnahmeeinrichtung und Verriegelungsschraube kann vorteilhaft ein mit der Zeit erfolgendes, unerwünschtes Heraustreten der Verriegelungsschraube entlang seiner Längsrichtung aus der Aufnahmeeinrichtung verhindern. Diese Einrichtungen zum Erhöhen der Reibung können dabei über lediglich einem Abschnitt (bzw. entlang des Abschnitts) der Aufnahmeeinrichtung und/ oder der Verriegelungsschraube oder deren gesamte Erstreckung vorgesehen sein.

Unter dem "Unterbinden" wird in manchen beispielhaften Ausführungsformen des erfindungsgemäßen Marknagels ein Beenden der Ausrichtbarkeit der in die Aufnahmeeinrichtung eingeführten Verriegelungseinrichtung, z. B. einer Verriegelungsschraube, im Raum, wenigstens in zumindest einer Raumrichtung oder einer Drehrichtung, verstanden. Dies kann durch ein Blockieren, ein Arretieren, ein Aushärten, ein Verriegeln oder dergleichen der Aufnahmeeinrichtung geschehen. Hierzu kann eine Einrichtung zum Unterbinden, wie eine oben beschriebene Klemmschraube, vorgesehen sein. Mit Unterbinden kann jedoch auch eine Wirkung oder Eigenschaft gemeint sein, die einer der Strukturen innewohnt. Als Beispiel wird hier die Aushärtbarkeit einer mittels Zement ausgestalteten Aufnahmeeinrichtung genannt.

Ein weiteres Beispiel, wie die Ausrichtbarkeit der Aufnahmeeinrichtung erfindungsgemäß unterbunden werden kann, ist das Vorsehen einer Einrichtung zur Erhöhung der Reibung zwischen Verriegelungseinrichtung und Knochennagel, wobei ein Einbringen der Verriegelungseinrichtung (bspw. durch Schrauben, Einschlagen, Einschieben oder dergleichen) in die Aufnahmeeinrichtung die Ausrichtbarkeit der Aufnahmeeinrichtung zunehmend oder schlagartig beendet. Dies kann durch ein Aufspreizen der Aufnahmeeinrichtung mittels des bspw. konisch zulaufenden Schafts der Verriegelungseinrichtung bewerkstelligt werden. Durch ein solches Aufspreizen oder auch nur eine entsprechende Druckerhöhung mit Verformung einer äußeren Kontur der Aufnahmeeinrichtung oder wenigstens Erhöhen des Drucks der Aufnahmeeinrichtung auf ihre Umgebung kann die Aufnahmeeinrichtung gegen eine weitere Struktur des Marknagels derart gedrückt werden, dass die Aufnahmestruktur gegenüber dem Marknagel in ihrer Bewegungsfreiheit und insbesondere in ihrer Ausrichtbarkeit blockiert ist. Sie kann also allein durch das Eintreiben der Verriegelungseinrichtung in sie hinein arretiert werden.

Die Verriegelungseinrichtung (bspw. in Stift- oder Schraubenform) kann dabei in einigen beispielhaften erfindungsgemäßen Ausführungsformen derart ausgestaltet sein, dass erst das Einbringen ihres zuletzt in die Aufnahmeeinrichtung einzubringenden Abschnitts die Blockierung bewirkt. Es kann die Verriegelungseinrichtung für eine solche Blockierung vorbereitet sein. Die Aufnahmeeinrichtung kann ebenfalls entsprechend ausgestaltet sein. Zudem können sowohl die Verriegelungseinrichtung als auch die Aufnahmeeinrichtung zum Erzielen der Blockierung bzw. Verriegelung ausgestaltet sein.

Alle erfindungsgemäßen Gegenstände können in einigen beispielhaften erfindungsgemäßen Ausführungsformen aus einer Typ II anodisierten Titanlegierung (Ti6A14V) für verbesserte biomechanische und biomedizinische Leistungen gefertigt sein.

Manche oder alle erfindungsgemäßen Ausführungsformen können einen, mehrere oder alle der oben und/oder im Folgenden genannten Vorteile aufweisen.

Mittels des offenbarten Verfahrens zum Verblocken von wenigstens einer Hülse eines Marknagels kann die Hülse derart verformt werden, dass die Verriegelungsschraube in der Hülse lagestabil geklemmt oder verklemmt wird. Damit kann vorteilhaft eine Relativbewegung der Verriegelungsschraube zur Hülse und/oder zum Marknagel vorteilhaft verhindert werden und eine mögliche Lockerung des Marknagels im Röhrenknochen unterbunden werden. Ein sogenanntes "Herauswandern" oder ein sogenanntes "back-out" der Verriegelungsschraube kann vorteilhaft verhindert werden.

Mittels einer Vormontage der Hülse (oder mehreren Hülsen) und von einem oder mehreren Stützkörpern in der Kassette kann die gesamte Montage des Verriegelungsmarknagels vorteilhaft vereinfacht werden. Diese Vereinfachung kann die gesamte Montagezeit vorteilhaft verkürzen.

Eine formschlüssige Verbindung oder eine wieder lösbare Verankerung eines Stützkörpers in dem Schaft des Marknagels oder in der Kassette mittels einer Nut-Steg-Verbindung kann ein unerwünschtes Mitdrehen oder eine ungewollte Verschiebung der Verriegelungsschraube in den Hülsen vorteilhaft verhindern. Damit kann das Operationsergebnis bei der Versorgung von Knochenfrakturen und/ oder von Versteifungen von Gelenken mittels erfindungsgemäßer Verriegelungsmarknägel vorteilhaft verbessert werden.

Die vorliegende Erfindung wird im Folgenden anhand der beigefügten Figuren, in welcher identische Bezugszeichen gleiche oder ähnliche Bauteile bezeichnen, exemplarisch erläutert. In den jeweils schematisch vereinfachten Figuren gilt:
- **Fig. 1**: zeigt einen Abschnitt eines erfindungsgemäßen Verriegelungsmarknagels in perspektivischer Darstellung;
- **Fig. 2**: zeigt einen kassettenförmigen Einsatz, zwei Aufnahmeeinrichtungen sowie zwei Stützkörper in perspektivischer Darstellung;
- **Fig. 3**: zeigt einen erfindungsgemäßen Verriegelungsmarknagel in perspektivischer Gesamtansicht und in zwei perspektivischen Detailansichten;
- **Fig. 4**: zeigt eine weitere Ausführungsform eines kassettenförmigen Einsatzes, zwei Aufnahmeeinrichtungen sowie zwei Stützkörpern in perspektivischer Darstellung;
- **Fig. 5**: zeigt eine Schnittdarstellung eines weiteren erfindungsgemäßen Verriegelungsmarknagels mit einem kassettenförmigen Einsatz, vier Aufnahmeeinrichtungen sowie vier Stützkörpern;
- **Fig. 6 a-g**: zeigen verschiedene Ausführungsformen des Stützkörpers;
- **Fig. 7**: zeigt einen Abschnitt eines weiteren erfindungsgemäßen Verriegelungsmarknagels in perspektivischer Darstellung;
- **Fig. 8**: zeigt einen Abschnitt des erfindungsgemäßen Verriegelungsmarknagels aus Fig. 7 in Schnittdarstellung mit einer Aufnahmeeinrichtung, einem Stützkörper und einem Inlay-Ring;
- **Fig. 9**: zeigt einen Abschnitt des erfindungsgemäßen Verriegelungsmarknagels aus Fig. 7 in Schnittdarstellung mit einer Aufnahmeeinrichtung, einem Stützkörper und einem Inlay-Stab;
- **Fig. 10**: zeigt die Anordnung der Fig. 9 in perspektivischer Darstellung;
- **Fig. 11**: zeigt die Anordnung der Fig. 9, jedoch mit einem modifizierten Inlay-Stab, in perspektivischer Darstellung;
- **Fig. 12**: zeigt eine Schnittdarstellung eines weiteren erfindungsgemäßen Verriegelungsmarknagels mit Stützkörpern, Aufnahmeeinrichtungen sowie weiteren Inlays;
- **Fig. 13**: zeigt eine perspektivische Ansicht in einer Teilschnittdarstellung eines weiteren erfindungsgemäßen Verriegelungsmarknagels; und
- **Fig. 14 bis 16**: zeigen Schnittdarstellungen von weiteren erfindungsgemäßen Verriegelungsmarknägeln.

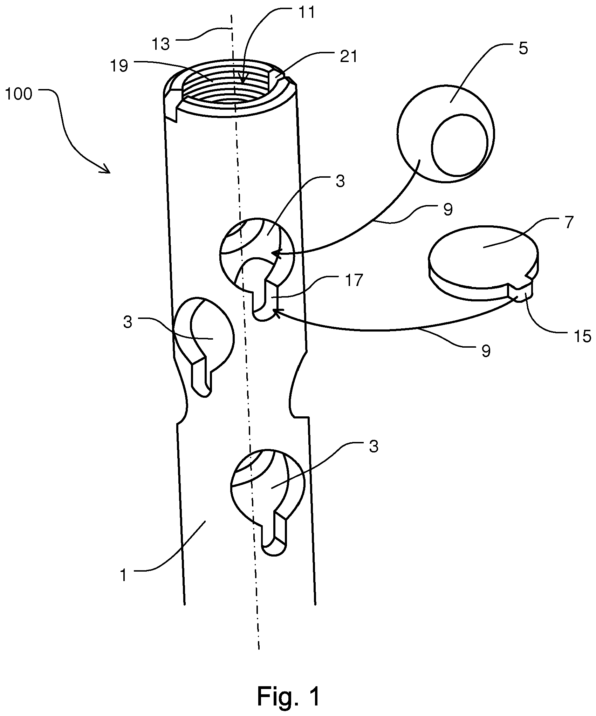

**Fig. 1** zeigt einen Abschnitt eines erfindungsgemäßen Verriegelungsmarknagels 100 in perspektivischer Darstellung. Die Einzelteile sind in Explosionsdarstellung gezeigt.

Der Abschnitt zeigt einen axialen Endbereich des Verriegelungsmarknagels 100. Der Verriegelungsmarknagel 100 weist einen länglichen Schaft 1 mit einem inneren Hohlraum, mehrere Öffnungen 3 zur Aufnahme von Verriegelungsvorrichtungen, eine Aufnahmeeinrichtung 5 zur Aufnahme einer Verriegelungsvorrichtung sowie einen Stützkörper 7 auf. Die Pfeile 9 deuten die Positionierung der Aufnahmeeinrichtung 5 und des Stützkörpers 7 im montierten Zustand an. Die Aufnahmeeinrichtung 5 und der Stützkörper 7 können durch die axiale Öffnung 11 in den Schaft 1 eingeführt werden. Insbesondere der Stützkörper 7 kann alternativ durch die Öffnung 3 direkt in den Schaft 1 eingefügt werden, indem der Stützkörper 7 zunächst gedreht, dann eingeführt und anschließend wieder gedreht und in die endgültige Position, in der die Oberfläche des Stützkörpers 7 senkrecht zur Längsachse 13 des Verriegelungsmarknagels 100 ausgerichtet ist, positioniert wird. In dieser Position ist ein Steg, Vorsprung oder Absatz 15 des Stützkörpers 7 formschlüssig mit einer Längsnut 17 (oder anderweitig ausgestalteten Aussparung, vorzugsweise in der Wandung) des Schafts 1 lösbar verbunden. Diese Verbindung kann als Nut-Steg-Verbindung bezeichnet werden. Die formschlüssige Verbindung kann eine Rotation des Stützköpers 7 um die Längsachse 13 des Verriegelungsmarknagels 100 und insbesondere eine Rotation der Aufnahmevorrichtung 5 verhindern, wenn der Stützköper 7 nach einer Montage direkt an der Aufnahmeeinrichtung 5 anliegt. Dies ist vorteilhaft, um eine ungewollte und unbeabsichtigte Rotation einer Verriegelungsvorrichtung (beispielsweise einer Verriegelungsschraube oder eines Verriegelungsstifts) in der Aufnahmeeinrichtung 5 zu verhindern. Eine derartige Rotation könnte zu einer Instabilität oder Lockerung eines fixierten Verriegelungsmarknagels 100 in einem Markraum eines Röhrenknochens führen.

Die Längsnut 17 oder anderweitige Aussparung kann vorzugsweise die Wandung des Schafts 1 öffnen, also eine Verbindung zwischen Innen und Außen, bezogen auf den Verriegelungsmarknagel 100, darstellen.

In Fig. 1 sind exemplarisch eine Aufnahmeeinrichtung 5 und ein Stützkörper 7 dargestellt. Weitere Aufnahmeeinrichtungen 5 und Stützkörper 7 können im Inneren des Schafts 1 hintereinander in Reihe (kaskadenförmig) angeordnet werden. Entsprechend können mehrere Verriegelungsvorrichtungen zur Fixierung des Verriegelungsmarknagels 100 im Markraum eines Röhrenknochens angeordnet werden.

Der oder die Aufnahmekörper 5 und der oder die Stützkörper 7 können im Inneren des Schafts 1 mittels einer Verschraubung am Schaftende verblockt werden. Das Schaftende kann dazu ein Innengewinde 19 aufweisen. Weiterhin kann das Innengewinde 19 wenigstens eine Längsnut 21 aufweisen, um den Absatz 15 eines Stützkörpers 7 aufzunehmen. Die Aufnahme des Stützkörpers 7 kann als formschlüssige Verbindung des Stützkörpers 7 mit dem Schaft 1 bezeichnet werden. Alternativ kann die Verschraubung direkt auf eine Aufnahmerichtung 5 einwirken, ohne direktem Anliegen eines Stützkörpers 7 an der Verschraubung. Zwischen der Verschraubung und dem Stützkörper 7 oder zwischen der Verschraubung und der Aufnahmeeinrichtung 5 können weitere Zwischenkörper (z. B. Distanzsegmente) angeordnet sein.

Mittels der Verschraubung können einzelne oder alle Aufnahmeeinrichtungen 5 verblockt werden. Mittels der Verblockung kann eine Rotation des Verriegelungsmarknagels 100 um die Längsachse 13 verhindert werden. Weiterhin kann die Verblockung Verriegelungsvorrichtungen in den Aufnahmeeinrichtungen 5 verblocken und verklemmen, so dass ein Verdrehen und Verschieben der Verriegelungsvorrichtungen in den Aufnahmeeinrichtungen 5 verhindert wird. Damit kann ein verschraubter Verriegelungsmarknagel 100 im Markraum eines Röhrenknochens vorteilhaft fixiert werden.

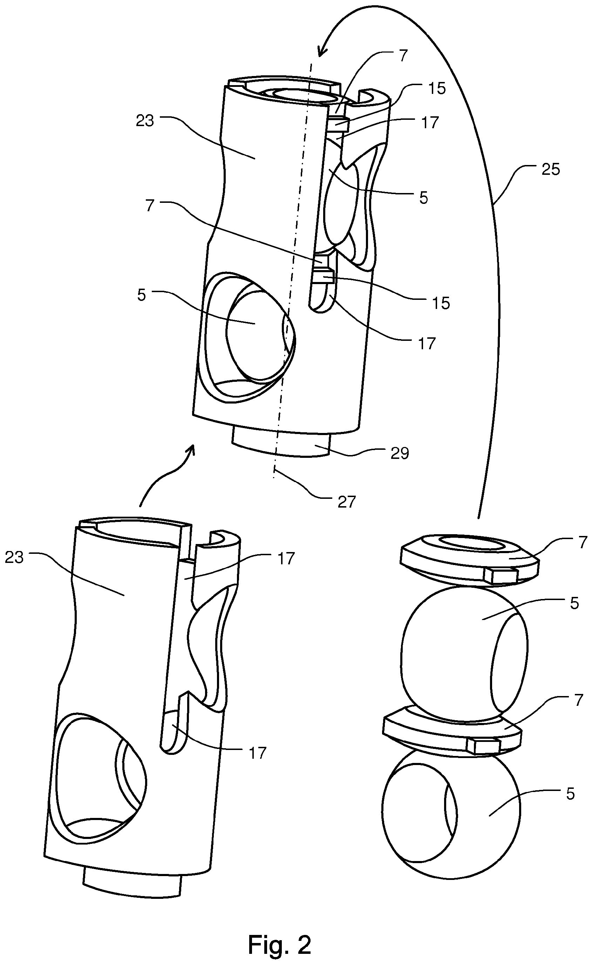

**Fig. 2** zeigt einen kassettenförmigen Einsatz 23, zwei Aufnahmeeinrichtungen 5 sowie zwei Stützkörper 7 in perspektivischer Darstellung, sowohl in Explosionsdarstellung, als auch in montiertem Zustand.

Der kassettenförmige Einsatz 23 kann, insbesondere im montierten Zustand (obere Abbildung in Fig. 2), in den Schaft 1 eines Verriegelungsmarknagels 100 eingeführt werden. Eine derartige Vormontage kann einerseits die Montage des Verriegelungsmarknagels 100 vereinfachen und erleichtern (separate Arbeitsschritte), andererseits die Ausrichtung und Positionierung der Aufnahmeeinrichtungen 5 und der Stützkörper 7 bereits bei der Montage des kassettenförmigen Einsatzes 23 festlegen. Die Montage erfolgt dabei durch schrittweises Einführen der im Folgenden beschriebenen Einzelteile in die obere Öffnung der Kassette 23 (Pfeilrichtung 25 zum Einführen der Einzelteile in die Kassette 23). Zunächst wird die erste Aufnahmeeinrichtung 5 (unterste Aufnahmeeinrichtung 5 in der rechten, unteren Abbildung in Fig. 2) in die Kassette 23 eingesetzt, anschließend der erste Stützkörper 7 (unterer Stützkörper 7 in der rechten, unteren Abbildung in Fig. 2), nachfolgend die zweite Aufnahmeeinrichtung 5 und der zweite Stützkörper 7. Die erste Aufnahmeeinrichtung 5 wird mit der Bohrung oder Öffnung der Aufnahmeeinrichtung 5 in Richtung der Öffnung in der Kassette 23 ausgerichtet, um ein späteres Einführen einer Verriegelungsvorrichtung in diese Durchführung zu vereinfachen. Anschließend wird der erste Stützkörper 7 mittels des Absatzes 15 rotationsausgerichtet in die Längsnut 17 der Kassette 23 eingeführt. Diese Nut-Steg-Verbindung verhindert ein nachfolgendes, unbeabsichtigtes Verdrehen des Stützkörpers 7 (um die Längsachse 27 der Kassette 23), insbesondere um ein nachfolgendes, unbeabsichtigtes Verdrehen der ersten Aufnahmeeinrichtung 5 zu verhindern. Ein Verdrehen der ersten Aufnahmeeinrichtung 5 wäre insbesondere nachteilig hinsichtlich eines zügigen Einbringens einer Verriegelungsvorrichtung bei einem Fixieren des Verriegelungsmarknagels 100 während einer Operation zur Versorgung von Knochenfrakturen.

Der Absatz 15 des zweiten Stützkörpers 7 kann alternativ zur Längsnut 17 in eine andere Längsnut am oberen Ende der Kassette 23 eingeführt werden. In Fig. 2 sind exemplarisch zwei weitere Längsnuten dargestellt.

Die Kassette 23 kann mittels eines Absatzes 29 (am unteren axialen Ende; entgegengesetzt der Öffnung zum Einbringen der Aufnahmeeinrichtungen 5 und der Stützkörper 7) im dem Schaft 1 des Verriegelungsmarknagels 100 formschlüssig positioniert und angeordnet werden. Insbesondere ist der Absatz 29 exzentrisch in Bezug auf die Längsachse 27 angeordnet, so dass die Position der Kassette 23 in dem Verriegelungsmarknagel 100 rotatorisch festgelegt und verdrehsicher ist. Alternativ zu dieser Verdrehsicherung kann die Kassette 23 einen Längssteg an der Außenseite der Kassette 23 aufweisen, der in eine Längsnut an der Innenseite des Schafts 1 eingeführt wird (formschlüssige Verbindung) und eine Verdrehung der Kassette 23 in dem Verriegelungsmarknagel 100 verhindert.

Die Form des Stützkörpers 7 ist in Fig. 2 rein exemplarisch dargestellt. In dieser Ausführungsform weist der Stützkörper 7 jeweils konkave Vertiefungen an den beiden Oberflächen auf, in denen die Aufnahmeeinrichtungen 5 anliegen.

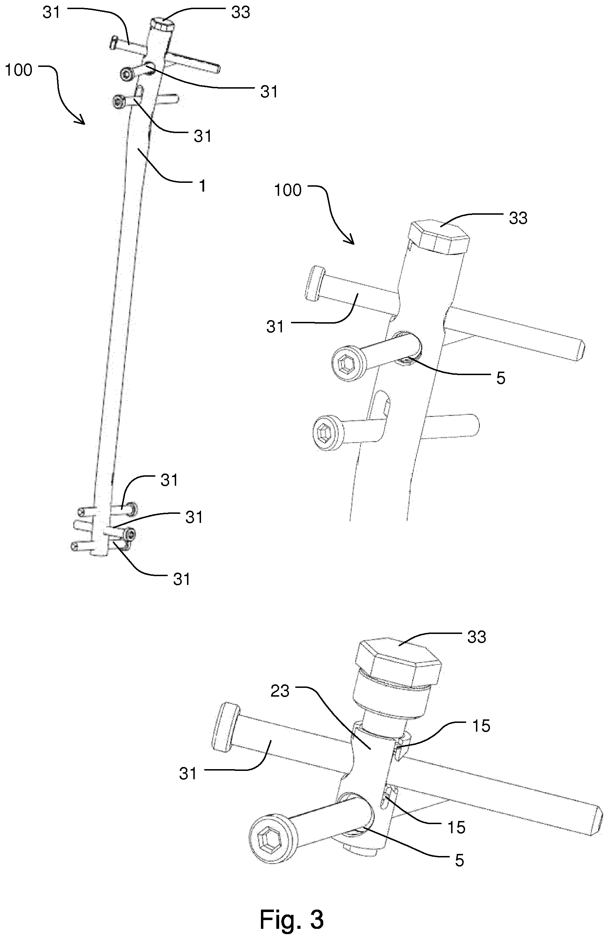

**Fig. 3** zeigt einen erfindungsgemäßen Verriegelungsmarknagel 100 in perspektivischer Gesamtansicht und in zwei perspektivischen Detailansichten.

Die Kassette 23 mit den Aufnahmeeinrichtungen 5 und den Stützkörpern 7 ist im oberen Endbereich des Verriegelungsmarknagels 100 (bezogen auf Fig. 3) angeordnet. Die beiden am oberen, axialen Endbereich des Verriegelungsmarknagels 100 angeordneten Verriegelungsschrauben 31 sind in den Aufnahmeeinrichtungen 5 der Kassette 23 positioniert und können, wenn sie nicht mittels einer Arretiervorrichtung 33 verblockt sind, innerhalb der Öffnungen in dem Schaft 1 des Verriegelungsmarknagels 100 gedreht und bewegt werden. Diese Verdrehmöglichkeit kann eine anatomisch vorteilhafte Fixierung der Verriegelungsschrauben 31 in einem Röhrenknochen erleichtern. Die weiteren Verriegelungsschrauben 31 sind als fixe Verriegelungsschrauben 31 ausgeführt, das heißt, die Verriegelungsschrauben 31 können nur innerhalb des Öffnungsbereichs im Schaft 1 bewegt werden, nicht jedoch innerhalb von Aufnahmeeinrichtungen 5 im dem Schaft 1 bewegt und gedreht werden.

Nach einer Ausrichtung der beiden oberen Verriegelungsschrauben 31 in den Aufnahmeeinrichtungen 5 können die Aufnahmeeinrichtungen 5 und die Stützkörper 7 in der Kassette 23 verblockt werden, beispielsweise mittels einer als Schraube ausgeführten Arretiervorrichtung 33 in einem Innengewinde 19 des Schaftendes (siehe Fig. 1). Die Verblockung kann zweistufig ausgeführt werden. In einer ersten Stufe kann die als Schraube ausgeführte Arretiervorrichtung 33 angezogen werden, so dass die Aufnahmeeinrichtungen 5 und die Stützkörper 7 in dem Schaft 1 nicht mehr bewegt werden können. In dieser ersten Stufe können die Verriegelungsschrauben 31 in den Aufnahmeeinrichtungen 5 weiterhin bewegt werden, beispielsweise entlang der Längsachse der Verriegelungsschrauben 31 verschoben werden. In einer zweiten Stufe kann die Arretiervorrichtung 33 stärker angezogen (oder festgezogen) werden, so dass die Aufnahmeeinrichtungen 5 derart verformt werden, dass die Verriegelungsschrauben 31 in den Aufnahmeeinrichtungen 5 festgeklemmt werden und nicht mehr bewegbar sind.

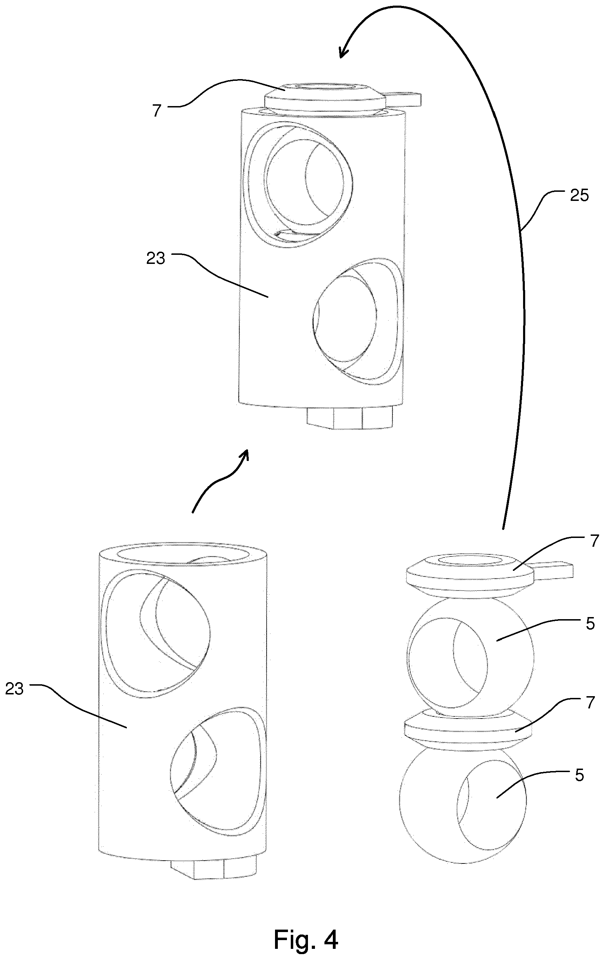

**Fig. 4** zeigt eine weitere Ausführungsform eines kassettenförmigen Einsatzes 23, zwei Aufnahmeeinrichtungen 5 sowie zwei Stützkörper 7 in perspektivischer Darstellung. Die obere Abbildung in Fig. 4 zeigt den montierten Zustand der in den unteren Abbildungen gezeigten Einzelteile (Explosionsdarstellung).

Im Gegensatz zur Fig. 2 weist die Kassette 23 in Fig. 4 keine Längsnut zum formschlüssigen Anordnen der Stützkörper 7 auf. Der untere Stützkörper 7, der in der unteren, rechten Abbildung gezeigt ist, weist folglich keinen Absatz für Längsnuten auf. Die Montage der Kassette 23 erfolgt weitgehend analog zur Fig. 2, ohne jedoch den ersten Stützkörper 7 (unterer Stützkörper 7) in einer Längsnut formschlüssig anzuordnen und ohne den zweiten Stützkörper 7 in der Kassette 23 anzuordnen. Der zweite Stützkörper 7 ist oberhalb der Kassette 7 angeordnet und kann in einer Längsnut des Schafts 1, beispielsweise in einer Längsnut in einem Gewinde am Schaftende (siehe Fig. 1, Längsnut 21 im Innengewinde 19) angeordnet sein. Mittels dieser Nut-Steg-Verbindung kann eine Rotationssicherung des Stützkörpers 7 erreicht werden. Damit wird wiederum eine ungewollte Rotation der an diesem Stützköper anliegenden Aufnahmeeinrichtung 5 erreicht werden.

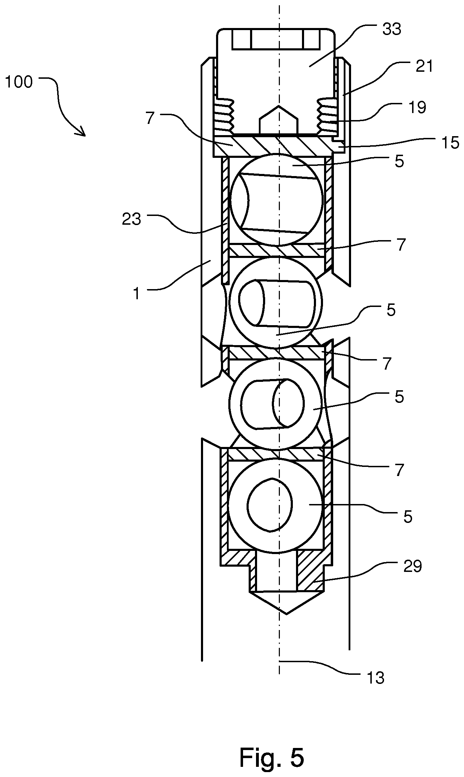

**Fig. 5** zeigt eine Schnittdarstellung eines weiteren erfindungsgemäßen Verriegelungsmarknagels 100 mit einem kassettenförmigen Einsatz 23, vier Aufnahmeeinrichtungen 5, vier Stützkörpern 7 sowie einer Innensechskantmutter als Arretiervorrichtung 33.

Die Kassette 23 in Fig. 5 ist eine Erweiterung der in Fig. 4 dargestellten Kassette 23. In Fig. 4 ist eine Kassette 23 mit zwei Aufnahmeeinrichtungen 5 und einem Stützkörper 7 in der Kassette 23 sowie einem weiteren Stützkörper 7 zwischen der Kassette 23 und der Arretiervorrichtung 33 dargestellt. In Fig. 5 ist eine Kassette 23 mit vier Aufnahmeeinrichtungen 5, drei Stützkörpern 7 in der Kassette 23 sowie einem weiteren Stützkörper 7 zwischen der Kassette 23 und der Arretiervorrichtung 33 dargestellt. Der Stützkörper 7 zwischen der Kassette 23 und der Arretiervorrichtung 33 weist einen Absatz 15 als Rotationssicherung auf, insbesondere um bei einem Verblocken der Kassette 23 mittels der Arretiervorrichtung 33 ein Verdrehen der Aufnahmeeinrichtungen 5 (und damit ein Verdrehen von Verriegelungsvorrichtungen (31) in den Aufnahmeeinrichtungen 5) zu verhindern. Der an der Arretiervorrichtung 33 anliegende Stützkörper 7 wird mit dem Absatz 15 durch die Längsnut 21 im Innengewinde des Schafts 1 eingeführt.

Die Ausrichtung der Kassette 23 relativ zum Schaft 1 erfolgt mittels des exzentrisch (in Bezug auf die Längsachse 13 des Verriegelungsmarknagels 100) angeordneten Absatzes 29 am axial unteren Ende der Kassette 23. Diese Ausrichtung ist insbesondere für die Ausrichtung der Durchgangsbohrungen der Aufnahmeeinrichtungen 5 mit den Öffnungen des Schafts 1 wichtig, um bei einem operativen Einsetzen des Verriegelungsmarknagels 100 in den Markraum eines Röhrenknochens die Verriegelungsvorrichtungen einfach und schnell einsetzen zu können.

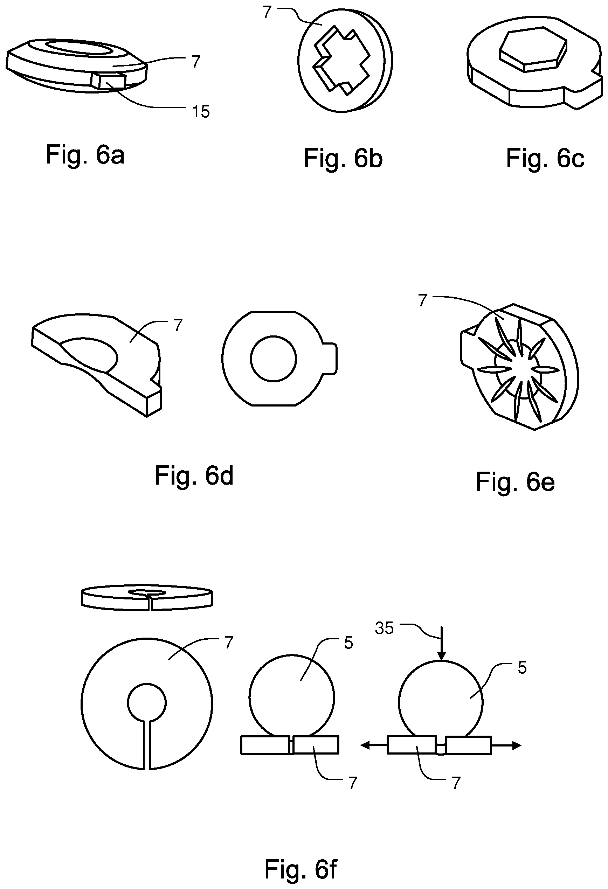

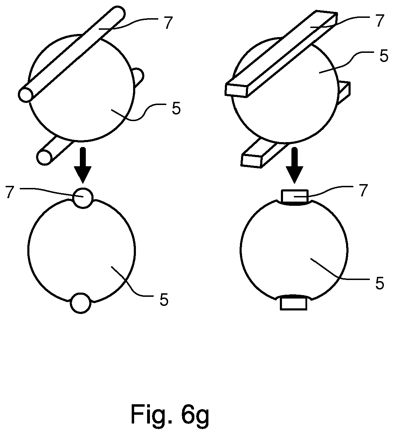

**Fig. 6 a-g** zeigen verschiedene Ausführungsformen des Stützkörpers 7. Die dargestellten Ausführungsformen sind rein exemplarisch und nicht auf die in Fig. 6a bis Fig. 6g gezeigten Ausführungsformen beschränkt.

Fig. 6a zeigt die Ausführungsform des Stützkörpers 7 aus Fig. 2. Der Stützkörper 7 weist einen Absatz 15 als Rotationssicherung für eine Nut-Steg-Verbindung auf. Weiterhin weist der Stützkörper 7 auf der Oberseite und Unterseite mittige, konkavförmige Vertiefungen zum formschlüssigen Anliegen der Aufnahmeeinrichtungen 5 auf. Die Oberflächen der Vertiefungen können zur Erhöhung des Reibwertes (zur Verbesserung der Rotationsstabilität anliegender Aufnahmeeinrichtungen 5) beispielsweise sandgestrahlt werden.

Fig. 6b zeigt eine Ausführungsform des Stützkörpers 7 mit einer lochförmigen Innenkontur und ohne einem Absatz 15 für eine Nut-Steg-Verbindung (siehe Fig. 6a). Die kantige Innenkontur kann die Rotationsstabilität anliegender Aufnahmeeinrichtungen 5 verbessern.

Fig. 6c zeigt eine Ausführungsform des Stützkörpers 7 mit einem sechskantigen, konvexen Absatz mittig auf der Oberfläche. Der sechskantige Absatz kann auch auf der gegenüberliegenden Oberfläche angeordnet sein. Für einen Kraftschluss und/oder Formschluss einer Aufnahmeeinrichtung 5 mit dem Stützkörper 7 kann sich die Aufnahmeeinrichtung 5 in den sechskantigen Absatz unter Krafteinwirkung einpressen.

Fig. 6d zeigt in einer Halbschnittdarstellung (linke Abbildung) und in einer Aufsicht (rechte Abbildung) eine ähnliche Ausführungsform des Stützkörpers 7 wie in Fig. 6a. Die Ausführungsform in Fig. 6c weist eine geringere Höhe oder Dicke als der Stützkörper 7 in Fig. 6a auf und somit eine geringe Bauhöhe. Dies kann beispielsweise für einen begrenzten Bauraum in einem Verriegelungsmarknagel 100 oder einer Kassette 23 vorteilhaft sein. Weiterhin weist der Stützkörper 7 eine nicht kreisförmige Form oder Außenkontur (abgeschnittene Kreissegmente oder Kreisabschnitte) auf. Dies kann als Montagehilfe bei einer entsprechenden Gegenform der Innenkontur der Kassette oder des Schafts vorteilhaft sein.

Fig. 6e zeigt eine weitere Ausführungsform des Stützkörpers 7 mit zwei abgeschnittenen oder abgetrennten Kreisabschnitten als mögliche Montagehilfen. Die sternförmige Innenkontur auf der Oberfläche kann die Rotationsstabilität anliegender Aufnahmeeinrichtungen 5 verbessern. Die sternförmige Innenkontur kann auch auf der gegenüberliegenden Oberseite angeordnet sein.

Fig. 6f zeigt eine ringförmige Kontur mit einem radialen Schlitz als Ausführungsform des Stützkörpers 7. Mittels einer einwirkenden Kraft 35 auf die Aufnahmeeinrichtung 5 wird der geschlitzte Stützkörper 7 auseinandergedrückt (in Fig. 6e durch die beiden horizontalen Pfeile angedeutet). Dadurch vergrößert sich der Außendurchmesser des ringförmigen Stützkörpers 7. Bei einem Anliegen des Stützkörpers 7 in der Kassette 23 oder im Schaft 1 im Ausgangszustand (mittlere Abbildung in Fig. 6e) wird durch die Einwirkung der Kraft 35 der Stützkörper 7 an die Innenseite der Kassette 23 oder des Schafts gedrückt. Durch diesen Kraftschluss der anliegenden Oberflächen kann eine Rotationssicherung der Aufnahmeeinrichtung 5, und damit auch einer Verriegelungsvorrichtung in der Aufnahmeeinrichtung 5 erreicht werden.

Fig. 6g zeigt anstatt der ringförmigen (Fig. 6b und Fig. 6f) oder scheibenförmigen (Fig. 6a, Fig. 6c, Fig. 6d und Fig. 6e) Stützkörper 7 stabförmige Stützkörper 7. Die Stabform ist in der linken Abbildung rund und in der rechten Abbildung rechteckig. In beiden Fällen werden die stabförmigen Stützkörper mittels Krafteinwirkung in die Aufnahmeeinrichtungen 5 eingedrückt oder eingepresst und somit ein Kraftschluss und/oder ein Formschluss der Oberflächen zur Erzielung einer Rotationssicherung erreicht.

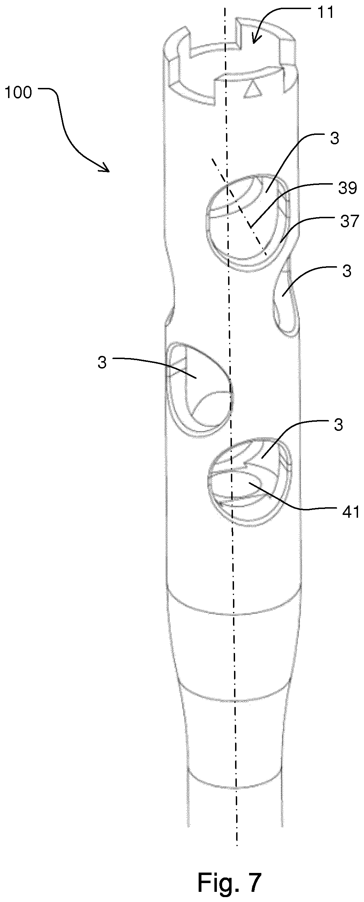

**Fig. 7** zeigt einen Abschnitt eines weiteren erfindungsgemäßen Verriegelungsmarknagels 100 in perspektivischer Darstellung mit vier Öffnungen 3 zur Aufnahme von Verriegelungsvorrichtungen, insbesondere Verriegelungsschrauben. Sowohl Aufnahmeeinrichtungen 5 als auch Stützkörper 7 (in Fig. 7 nicht dargestellt) können durch die axiale Öffnung 11 des Schafts in den Hohlraum des Verriegelungsmarknagels 100 eingeführt werden. Die Öffnungen 3 weisen am Umfang des äußeren Querschnitts Abschrägungen 37 auf, welche als Senkungen oder Fasen bezeichnet werden. Mittels dieser Abschrägungen 37 können Verriegelungsschrauben 31 in Hülsen 5 im Bereich der Öffnung (siehe Fig. 3) um eine Mittelachse 39 in einem größeren Winkelbereich bewegt werden als bei Öffnungen 3 ohne die Abschrägungen 37. Dadurch kann die Fixierung von Verriegelungsschrauben 31 im Verriegelungsmarknagel 100 und im Knochen vorteilhaft in einem größeren Verstellbereich erfolgen.

Der Verriegelungsmarknagel 100 weist am distalen Ende (in Fig. 7 unten) des inneren Hohlraums des Verriegelungsmarknagels 100 eine Vertiefung 41 (die Vertiefungen können als Mulden oder Aussparungen bezeichnet werden; sie sind Durchgangsöffnungen oder keine Durchgangsöffnungen) auf.

Die Oberfläche der Vertiefung 41 kann eine gleiche oder ähnliche Oberflächenform und/oder Oberflächenbeschaffenheit aufweisen wie die weiter oben beschriebenen Stützkörper 7, insbesondere in den Fig. 6a bis 6f. Die Vertiefung 41 kann funktional in der gleichen Weise wirken wie die Stützkörper 7, also beispielsweise als erhöhter Reibwiderstand gegen eine unbeabsichtigte Verdrehung oder Rotation der in Fig. 7 nicht gezeigten Hülse 5.

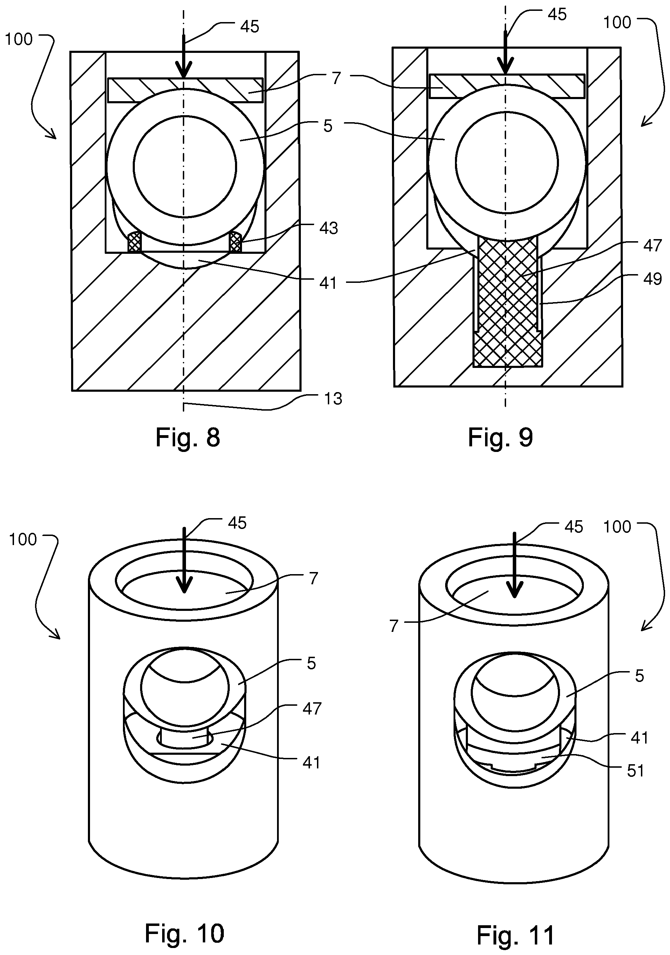

**Fig. 8** zeigt einen Abschnitt des erfindungsgemäßen Verriegelungsmarknagels 100 aus Fig. 7 in Längsschnittdarstellung mit einer Aufnahmeeinrichtung 5 (Hülse), einem Stützkörper 7 und einem Inlay-Ring 43 (welcher nur optional als Ring ausgestaltet ist). Der Inlay-Ring 43 ist zwischen der Hülse 5 und der Vertiefung 41 angeordnet. Mittels einer axialen Kraft 45 auf den Stützkörper 7 (dargestellt durch einen Pfeil) kann die Aufnahmeeinrichtung 5 auf oder gegen den Inlay-Ring 43 gedrückt werden. Der Inlay-Ring 43 ist vorzugsweise aus einem elastisch verformbaren Material hergestellt oder weist ein solches auf. Das Material des Inlay-Rings 43 kann ein Kunststoff sein, beispielsweise Polyetheretherketon (abgekürzt PEEK).

Bei Aufbringen oder Anlegen einer axialen Kraft 45 auf den Stützkörper 7 kann eine Vorspannung auf den Stützkörper 5 erzeugt werden. Der Inlay-Ring 43 kann als Gegenlager zu dieser axialen Kraft 45 wirken. Mittels dieser Vorspannung kann eine Transportsicherung generiert werden, wenn die Aufnahmeeinrichtung 5 (und gegebenenfalls weitere Aufnahmeeinrichtungen 5) vor einem Transport ausgerichtet wird, und diese Ausrichtung als Ausgangszustand zum Fixeren eines Marknagels in einem Röhrenknochen, als Operationshilfe für einen zügigen Operationsablauf, erhalten bleiben soll. Bei einer weiteren Erhöhung der axialen Kraft 45 über die Vorspannung hinaus können der Stützkörper 7 und die Hülse 5 weiter axial verschoben und der Inlay-Ring 43 soweit verformt werden, bis die Aufnahmeeinrichtung 5 in die Mulde 41 hineingedrückt wird und ein Formschluss zwischen den beiden Oberflächen entsteht. Dieser Formschluss kann dann als Verblockung der Aufnahmeeinrichtung 5 im Verriegelungsmarknagel 100 bezeichnet werden.

Wie Fig. 8 zu entnehmen ist, kann die Vertiefung die Form eines Ausschnitts einer Kugeloberfläche haben. Sie kann auf andere Weise in ein, zwei oder mehr Richtungen gekrümmt sein.

**Fig. 9** zeigt einen Abschnitt des erfindungsgemäßen Verriegelungsmarknagels 100 aus Fig. 7 in Schnittdarstellung mit einer Aufnahmeeinrichtung 5 (Hülse), einem Stützkörper 7 und einem Inlay-Stab 47. Der Inlay-Stab 47 wird in gleicher oder ähnlicher Weise verwendet wie der Inlay-Ring 43 aus Fig. 8. Der Inlay-Stab 47 kann aus dem gleichen Material hergestellt sein oder dieses aufweisen wie der Inlay-Ring 43. Die Vorspannung kann analog zu der Beschreibung der Fig. 8 aufgebaut werden. Bei einer weiteren Erhöhung der axialen Kraft 45 über die Vorspannung hinaus können der Stützkörper 7 und die Hülse 5 weiter axial verschoben und der Inlay-Stab 47 soweit verformt oder gestaucht werden (und sich in den Spalt 49 hinein verformen), bis die Aufnahmeeinrichtung 5 in die Mulde 41 hineingedrückt wird und ein Formschluss zwischen den Oberflächen entsteht. Dieser Formschluss kann dann als Verblockung der Aufnahmeeinrichtung 5 im Verriegelungsmarknagel 100 bezeichnet werden.

**Fig. 10** zeigt die Anordnung der Fig. 9 in perspektivischer Darstellung.

**Fig. 11** zeigt die Anordnung der Fig. 9, jedoch mit einem modifizierten Inlay-Stab 51 mit einer gegenüber dem Inlay-Stab 47 der Fig. 9 vergrößerten Auflagefläche zur Auflage der Hülse 5 hierauf mit dem Vorteil erhöhter Haftreibung in perspektivischer Darstellung. Im Übrigen kann das zu Fig. 9 Gesagte auch hier zutreffen.

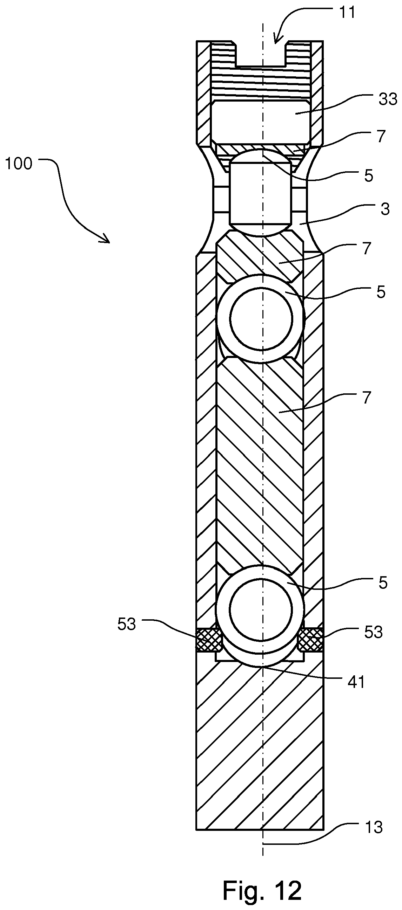

**Fig. 12** zeigt eine Schnittdarstellung eines weiteren erfindungsgemäßen Verriegelungsmarknagels 100 mit Stützkörpern 7, Aufnahmeeinrichtungen 5 (Hülsen) sowie weiteren Inlays. Die zylinderförmigen Inlays 53 sind gegenüber den Anordnungen aus den Fig. 8 bis 11 in Fig. 12 radial in dem Schaft des Verriegelungsmarknagels 100 angeordnet. Die zylinderförmigen oder ringförmigen Inlays 53 ragen in den inneren Hohlraum des Verriegelungsmarknagels 100 hinein und verhindern ein Durchrutschen der unteren (distalen) Hülse 5 in die Mulde 41. Die zylinderförmigen Inlays 53 wirken wie der Inlay-Ring 43 in Fig. 8 und der Inlay-Stab 47 in den Fig. 9 bis 11. Bei Aufbringen oder Anlegen einer axialen Kraft mittels der Arretiervorrichtung 33 (die beispielsweise als Madenschraube ausgeführt sein kann), etwa auf den oberen (proximalen) Stützkörper 7, kann eine Vorspannung oder ein Formschluss auf die Aufnahmeeinrichtungen 5 erzeugt oder ausgeübt werden. Die zylinderförmigen Inlays 53 können als Gegenlager zu dieser axialen Kraft wirken. Bei einer Erhöhung der axialen Kraft über die Vorspannung hinaus werden die zylinderförmigen Inlays 53 nach radial außen gedrückt oder gepresst, so dass die distale Hülse 5 weiter nach unten verschoben werden kann. Die Verschiebung kann bis zum Hineindrücken der Hülse 5 in die Mulde 41 und einem Formschluss der betroffenen Oberflächen erfolgen. Dieser Formschluss kann dann als Verblockung der Aufnahmeeinrichtung 5 im Verriegelungsmarknagel 100 bezeichnet werden.

Die exemplarisch als Madenschraube ausgeführte Arretiervorrichtung 33 kann beispielsweise mit einem Drehmoment von ca. 0,2 Nm angezogen werden, um damit eine Axialkraft, wie oben beschrieben, auf die Hülsen 5 und auf die Stützkörper 7 auszuüben. Mittels dieser Axialkraft können insbesondere die Hülsen 5 vorgespannt werden, so dass die Hülsen 5 zumindest rotationsstabil zwischen den Stützkörpern 7 fixiert sind. Eine derartige Fixierung kann vorteilhaft sein, um die Hülsen 5 vor einem Einbringen des erfindungsgemäßen Verriegelungsmarknagels 100 in den Markraum eines Röhrenknochens auszurichten. Diese Ausrichtung bleibt dann während des Einbringens des Verriegelungsmarknagels 100 in den Markraum des Röhrenknochens lagestabil fixiert und kann anschließend, ohne einer erneuten Ausrichtung der Hülsen 5, mittels Verriegelungsschrauben 31 in einem Röhrenknochen fixiert werden. Die Vorspannung wird insbesondere derart mittels eines geeigneten Drehmoments gewählt oder ausgeübt, dass die Hülsen 5, trotz einer lagestabilen oder rotationstabilen Fixierung, nach Einbringung in den Markraum des Röhrenknochens erneut zumindest geringfügig in ihrer Lage verändert und an anatomische Gegebenheiten angepasst werden können.

Die axiale Verschiebung der Hülsen 5 und der Stützkörper 7 aufgrund einer axialen Krafteinwirkung mittels der Arretiervorrichtung 33 kann rein exemplarisch beispielsweise 1 bis 2 mm betragen.

Das aufzubringende Drehmoment kann konstruktionsbedingt auf einen bevorzugten Wert von zwischen 0,05 Nm und 0,5 Nm, besonders bevorzugt zwischen 0,1 und 0,3 Nm und ganz besonders bevorzugt von ca. 0,2 Nm festgelegt sein.

Das oder die zylinderförmigen Inlays 53 können in unterschiedlicher Anzahl und Anordnung in dem Schaft des Verriegelungsmarknagels 100 angeordnet sein. Beispielsweise kann eine Anordnung ein, zwei, drei, vier oder mehr Inlays 53 aufweisen. Die Inlays 53 können radial, senkrecht zur Längsachse 13, oder in einem anderen Winkel, zum Beispiel tangential in Umfangsrichtung des Verriegelungsmarknagels 100 angeordnet sein.

Die zylinderförmigen Inlays 53 können eine Stabform, eine Balkenform oder eine andere Form aufweisen.

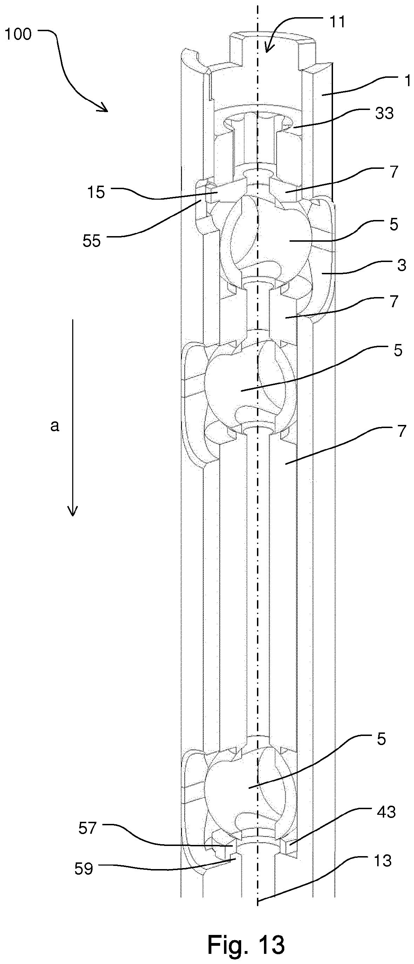

**Fig. 13** zeigt eine perspektivische Ansicht in einer Teilschnittdarstellung eines weiteren erfindungsgemäßen Verriegelungsmarknagels 100. Der Aufbau des Verriegelungsmarknagels 100 der Fig. 13 ist ähnlich dem Aufbau des in Fig. 12 dargestellten Verriegelungsmarknagels 100.

In den inneren Hohlraum des länglichen Schafts 1 wurden mehrere Bauteile durch die axiale Öffnung 11 modular eingebracht oder eingeschoben. In der exemplarischen Ausführungsform der Fig. 13 weisen alle (alternativ nicht alle) eingeführten Bauteile eine innere, axiale Öffnung oder Bohrung auf. Auf diese Weise ergibt sich eine Führungsdrahtöffnung, über welche ein Führungsdraht über alle sich aneinanderreihenden inneren axialen Öffnungen oder Bohrungen ergibt. Die Längsachse 13 ist in den Fig. 13 bis Fig. 16 inmitten der Führungsdrahtöffnung eingezeichnet. Die Vorteile, die mit der Möglichkeit einen Führungsdraht einführen zu können, ergeben, sind dem Fachmann bekannt.

In Fig. 13 ist erkennbar, dass diese innere Öffnung oder Bohrung durchgängig von oben (im Bereich der axialen Öffnung 11) bis zum unteren Ende des Schafts 1, zumindest aber des Hohlraums, verläuft ("oben" und "unten" bezieht sich im Zweifel auf die Darstellung der Figuren, hier der Fig. 13).

Die Öffnungen oder Bohrungen der einzelnen Bauteile können als Kanülierungen bezeichnet werden. Eine durchgängige Kanülierung ist mit den für den Fachmann im Zusammenhang mit der Kanülierung bekannten Vorteilen nutzbar. Beispielsweise kann die Kanülierung im Rahmen einer Spickdrahtosteosynthese genutzt werden.

Den oberen Abschluss der eingebrachten Bauteile bildet hier rein exemplarisch die Arretiervorrichtung 33, die optional mit einem Aussengewinde in den Schaft 1 eingeschraubt wurde. Zum Einschrauben der Arretiervorrichtung 33 ist exemplarisch innen ein Schrauben-Mitnahmeprofil in Vielrundform (sogenannte "Torx"-Form) dargestellt, das axial durchgängig offen ausgeführt ist.

Das im Beispiel der Fig. 13 der Arretiervorrichtung 33 in Axialrichtung a nächstliegende Bauteil ist ein erster Stützkörper 7, welche wiederum mit einer inneren Bohrung oder Öffnung versehen ist.

Zusätzlich weist der erste Stützkörper 7 optional am äußeren Umfang einen Absatz 15 oder Vorsprung auf. Dieser Absatz 15 greift als Verdrehsicherung in eine Nut, Vertiefung oder Längsnut 55 des Schafts 1 ein. Die Längsnut 55 ist hier exemplarisch als nach außen durchgebrochene Nut ausgeführt.

Die in Axialrichtung a auf den Stützkörper 7 nachfolgenden Bauteile sind eine erste Aufnahmeeinrichtung 5, ein optionaler zweiter Stützkörper 7, gefolgt von einer optionalen zweiten, um (beispielsweise) 90 Grad (bezogen auf die Achsrichtung a) gedrehten Aufnahmeeinrichtung 5, einem dritten, in Achsrichtung a verglichen mit dem ersten oder dem zweiten Stützkörper deutlich längeren, optionalen Stützkörper 7 sowie einer optionalen dritten Aufnahmeeinrichtung 5. Alle vorstehend genannten Bauteile sind optional und weisen optional eine innere Kanülierung auf.

Am unteren, distalen, Ende des Verriegelungsmarknagels 100 oder seiner inneren Bohrung oder Öffnung ist optional ein Inlay-Ring 43 angeordnet, ähnlich dem Inlay-Ring 43 aus Fig. 8, hier jedoch mit einer inneren Bohrung oder Öffnung und einem Schlitz 57. Mittels des Schlitzes 57, der an einem optionalen Steg oder Absatz 59 anliegen kann, wird eine Verdrehsicherung des Inlay-Ring 43 erreicht. Der Verriegelungsmarknagel 100 ist im weiteren, distalen Bereich vorzugsweise ebenfalls kanüliert.

Das Material des Inlay-Rings 43 kann ein Kunststoff sein oder aufweisen, beispielsweise Polyetheretherketon (abgekürzt PEEK).

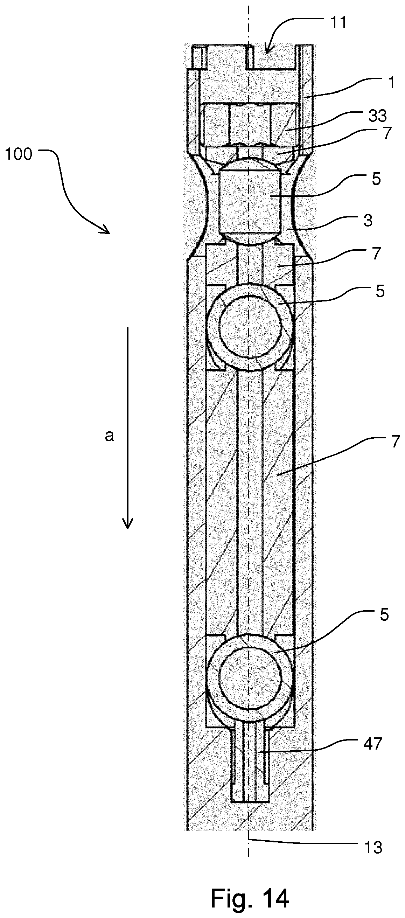

**Fig. 14** zeigt eine Schnittdarstellung eines weiteren erfindungsgemäßen Verriegelungsmarknagels 100. Die Stützkörper 7 sowie der Inlay-Stab 47 (siehe Fig. 9) sind kanüliert. Die Kanülierung des Inlay-Stabs 47 kann vorteilhaft genutzt werden, um eine Stauchung und Verformung des Inlay-Stabs 47 mit einer geringeren Kraft oder einem geringeren Druck gegenüber einem Inlay-Stab 47 ohne Kanülierung zu erreichen. Mittels einer Stauchung und/oder Verformung kann eine Verblockung wenigstens der distalen (in Fig. 14 unteren) Aufnahmeeinrichtung 5 im Verriegelungsmarknagel 100 erreicht werden.

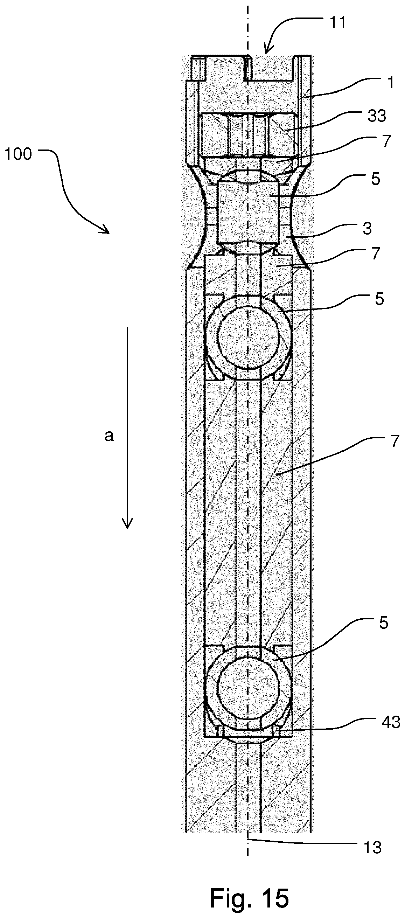

**Fig. 15** zeigt eine Schnittdarstellung eines weiteren erfindungsgemäßen Verriegelungsmarknagels 100. Gegenüber dem Verriegelungsmarknagel 100 aus Fig. 14 ist die Kanülierung der in den länglichen Schaft 1 des inneren Hohlraums eingebrachten oder eingeschobenen Bauteile sowie des distalen Bereichs des Verriegelungsmarknagels 100 durchgängig kanüliert. Die Verblockung der wenigstens distalen (unteren) Aufnahmeeinrichtung 5 erfolgt mittels eines Inlay-Rings 43 bzw. gegen diesen.

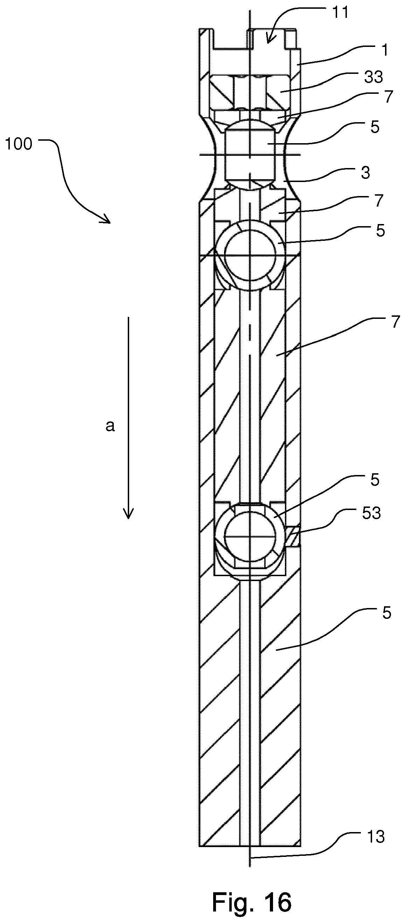

**Fig. 16** zeigt eine Schnittdarstellung eines weiteren erfindungsgemäßen Verriegelungsmarknagels 100. Gegenüber dem Verriegelungsmarknagel 100 aus Fig. 15 erfolgt die Verblockung der distalen (unteren) Aufnahmeeinrichtung 5 nicht mittels eines Inlay-Rings, sondern gegen ein radial eingeführtes, zylinderförmiges Inlay 53 (Inlaystab). Gegenüber den zylinderförmigen Inlays 53 in Fig. 12 erfolgt die Verblockung oder Rotationssicherung mittels eines (und nicht zwei, also anders als in Fig. 12) axial mittig (und nicht am distalen Ende wie in Fig. 12) angeordneten zylinderförmigen Inlays 53.

### Bezugszeichenliste

- 100: Verriegelungsmarknagel
- a: Axialrichtung, Längsrichtung
- 1: Schaft
- 3: Öffnung (zur Aufnahme von Verriegelungsvorrichtungen)
- 5: Aufnahmeeinrichtung; Hülse
- 7: Stützkörper
- 9: Pfeil; Positionierung der Aufnahmeeinrichtung und des Stützkörpers
- 11: axiale Öffnung des Schafts
- 13: Längsachse des Verriegelungsmarknagels
- 15: Absatz des Stützkörpers; Steg
- 17: Längsnut im kassettenförmigen Einsatz
- 19: Innengewinde
- 21: Längsnut im Innengewinde
- 23: kassettenförmiger Einsatz, Kassette
- 25: Pfeilrichtung der Montage der Kassette
- 27: Längsachse der Kassette
- 29: Absatz der Kassette
- 31: Verriegelungsschraube (Verriegelungsvorrichtung)
- 33: Arretiervorrichtung
- 35: einwirkende Kraft auf die Aufnahmeeinrichtung
- 37: Abschrägung, Fase
- 39: Mittelachse der Öffnung zum Aufnehmen von Verriegelungsvorrichtungen
- 41: Vertiefung, Mulde, Aussparung
- 43: Inlay-Ring
- 45: axiale Kraft
- 47: Inlay-Stab
- 49: Spalt
- 51: modifizierter Inlay-Stab
- 53: zylinderförmiges Inlay
- 55: Längsnut
- 57: Schlitz
- 59: Absatz, Steg

## Patentansprüche

1. Verriegelungsmarknagel (100) zum Einbringen in den Markraum eines Röhrenknochens zur Versorgung von Knochenfrakturen und/ oder zur Versteifung von Gelenken, mit einem länglichen Schaft (1) mit wenigstens einer Öffnung (3) zur Aufnahme von wenigstens einer Verriegelungsvorrichtung, und wenigstens einer Aufnahmeeinrichtung (5) zur Aufnahme der Verriegelungsvorrichtung, wobei die Aufnahmeeinrichtung (5) im Bereich der Öffnung (3) angeordnet ist, und wenigstens einem Stützkörper (7), welcher in Kontakt mit wenigstens einer Aufnahmeeinrichtung (5) angeordnet ist,
**dadurch gekennzeichnet, dass**
der Stützkörper (7) wenigstens abschnittsweise formschlüssig mit dem länglichen Schaft (1) und/ oder wenigstens abschnittsweise formschlüssig mit einem kassettenförmigen Einsatz (23), welcher in dem länglichen Schaft (1) positionierbar ist, lösbar verbunden ist, und dass der formschlüssig mit dem länglichen Schaft (1) oder der formschlüssig mit dem kassettenförmigen Einsatz (23) verbundene Stützkörper (7) zur Rotationssicherung der in der Aufnahmeeinrichtung (5) angeordneten Verriegelungsvorrichtung vorgesehen ist.

2. Verriegelungsmarknagel (100) nach Anspruch 1, wobei der Stützkörper (7) mittels einer Nut-Steg-Verbindung mit dem länglichen Schaft (1) und/ oder mit dem kassettenförmigen Einsatz (23) lösbar verbunden ist oder abschnittsweise in eine Nut eingeführt ist.

3. Verriegelungsmarknagel (100) nach Anspruch 1 oder 2, wobei der Stützkörper (7) auf seinem äußeren Umfang wenigstens einen Absatz, Vorsprung oder Steg (15) aufweist.

4. Verriegelungsmarknagel (100) nach einem der vorangegangenen Ansprüche, wobei der Schaft (1) des Verriegelungsmarknagels (100) und/oder der/ein kassettenförmige(r) Einsatz (23) wenigstens eine Öffnung (3) zur Aufnahme der Verriegelungsvorrichtung mit wenigstens einer Nut (17) oder Aussparung zur Aufnahme des Stützkörpers (7) aufweist.

5. Verriegelungsmarknagel (100) nach einem der vorangegangenen Ansprüche, wobei der Stützkörper (7) zwischen zwei aufeinander folgenden oder einander benachbarten Aufnahmeeinrichtungen (5) angeordnet ist.

6. Verriegelungsmarknagel (100) nach einem der vorangegangenen Ansprüche, wobei der Stützkörper (7) scheibenförmig ausgestaltet ist und in einem zentralen Bereich eine konvexe oder konkave Profilierung aufweist.

7. Verriegelungsmarknagel (100) nach einem der vorangegangenen Ansprüche, wobei der Stützkörper (7) ringförmig ausgestaltet ist und ein in einem zentralen Bereich hiervon offenes Profil aufweist.

8. Verriegelungsmarknagel (100) nach einem der Ansprüche 1 bis 5, wobei der Stützkörper (7) eine stabförmige Kontur aufweist.

9. Verriegelungsmarknagel (100) nach einem der vorangegangenen Ansprüche, wobei die Aufnahmeeinrichtung (5) aus einem ersten Material gefertigt ist oder dieses aufweist, und wobei die Aufnahmeeinrichtung (5) wenigstens einen Abschnitt aus einem zweiten, vom ersten Material verschiedenen, Material aufweist.

10. Verriegelungsmarknagel (100) nach einem der vorangegangenen Ansprüche, wobei die Aufnahmeeinrichtung (5) vollständig oder zumindest abschnittsweise beschichtet ist.

11. Verriegelungsmarknagel (100) nach einem der vorangegangenen Ansprüche, wobei die Aufnahmeeinrichtung (5) wenigstens einen Gewindegang zur Aufnahme einer Verriegelungsvorrichtung mit einem Gewindeabschnitt aufweist.

12. Verriegelungsmarknagel (100) nach einem der vorangegangenen Ansprüche, wobei der Verriegelungsmarknagel (100) wenigstens eine Arretiervorrichtung (33) zum Verblocken der wenigstens einen Aufnahmeeinrichtung (5) aufweist.

13. Verriegelungsmarknagel (100) nach einem der vorangegangenen Ansprüche, wobei der Verriegelungsmarknagel (100) eine Exzentereinrichtung zum Verblocken der wenigstens einen Aufnahmeeinrichtung (5) aufweist, wobei die Exzentereinrichtung vorzugsweise eine elastische Gegenkraft nach Aufbringen einer Kraft zum Verblocken der Aufnahmeeinrichtung (5) mittels einer Arretiervorrichtung (33) erzeugt.

14. Verriegelungsmarknagel (100) nach einem der vorangegangenen Ansprüche, wobei die wenigstens eine Aufnahmeeinrichtung (5) wenigstens eine durch sie hindurchreichende Führungsdrahtöffnung zum Aufnehmen eines Führungsdrahts aufweist.

15. Verriegelungsmarknagel (100) nach einem der vorangegangenen Ansprüche, wobei wenigstens einer der Stützkörper (7) wenigstens eine Führungsdrahtöffnung aufweist.

## Claims

1. A locking intramedullary nail (100) for insertion into the medullary cavity of a tubular bone for the treatment of bone fractures and/or for stiffening joints, comprising an elongated shaft (1) having at least one opening (3) for receiving at least one locking apparatus, and at least one receiving device (5) for receiving the locking apparatus, wherein the receiving device (5) is arranged in the region of the opening (3), and at least one supporting body (7) arranged in contact with at least one receiving device (5),
**characterized in that**
the supporting body (7) is, at least in sections, detachably connected in a positive-locking manner to the elongated shaft (1) and/or, at least in sections, detachably connected in a positive-locking manner to a cassette-shaped insert (23), which can be positioned in the elongated shaft (1),
and **in that** the positively-locked supporting body (7) connected to the elongated shaft (1) or to the cassette-shaped insert (23) is provided for rotationally securing the locking apparatus arranged in the receiving device (5).

2. The locking intramedullary nail (100) according to claim 1, wherein the supporting body (7) is detachably connected to the elongated shaft (1) and/or to the cassette-shaped insert (23) by means of a groove and ridge connection or is partially inserted into a groove.

3. The locking intramedullary nail (100) according to claim 1 or 2, wherein the supporting body (7) comprises at least one ledge, protrusion or ridge (15) on its outer periphery.

4. The locking intramedullary nail (100) according to anyone of the preceding claims, wherein the shaft (1) of the locking intramedullary nail (100) and/or the or one cassette-shaped insert (23) comprises/comprise at least one opening (3) for receiving the locking apparatus with at least one groove (17) or recess for receiving the supporting body (7).

5. The locking intramedullary nail (100) according to anyone of the preceding claims, wherein the supporting body (7) is arranged between two successive or adjacent receiving devices (5).

6. The locking intramedullary nail (100) according to anyone of the preceding claims, wherein the supporting body (7) is disc-shaped and has a convex or concave profile in a central region.

7. The locking intramedullary nail (100) according to anyone of the preceding claims, wherein the supporting body (7) is ring-shaped and has an open profile in a central region thereof.

8. The locking intramedullary nail (100) according to anyone of claims 1 to 5, wherein the supporting body (7) has a rod-shaped contour.

9. The locking intramedullary nail (100) according to anyone of the preceding claims, wherein the receiving device (5) is made of, or comprises, a first material, and wherein the receiving device (5) comprises at least one portion of a second material being different from the first material.

10. The locking intramedullary nail (100) according to anyone of the preceding claims, wherein the receiving device (5) is completely or at least partially coated.

11. The locking intramedullary nail (100) according to anyone of the preceding claims, wherein the receiving device (5) comprises at least one thread for receiving a locking apparatus having a thread portion.

12. The locking intramedullary nail (100) according to anyone of the preceding claims, wherein the locking intramedullary nail (100) comprises at least one arresting apparatus (33) for blocking the at least one receiving device (5).

13. The locking intramedullary nail (100) according to anyone of the preceding claims, wherein the locking intramedullary nail (100) comprises an eccentric device for blocking the at least one receiving device (5), the eccentric device preferably generating an elastic counter force after application of a force for blocking the receiving device (5) by means of an arresting apparatus (33).

14. The locking intramedullary nail (100) according to anyone of the preceding claims, wherein the at least one receiving device (5) comprises at least one guide wire opening passing through it for receiving a guide wire.

15. The locking intramedullary nail (100) according to anyone of the preceding claims, wherein at least one of the supporting bodies (7) comprises at least one guide wire opening.

## Revendications

1. Un clou intramédullaire de verrouillage (100) destiné à être inséré dans la cavité médullaire d'un os tubulaire pour le traitement de fractures osseuses et/ou pour rigidifier des articulations, comprenant une tige allongée (1) avec au moins un orifice (3) pour recevoir au moins un appareil de verrouillage, au moins un dispositif de réception (5) pour recevoir l'appareil de verrouillage, le dispositif de réception (5) étant agencé dans la région de l'orifice (3), et au moins un corps de support (7) disposé en contact avec au moins un dispositif de réception (5),
**caractérisé en ce que**
le corps de support (7) est relié de manière amovible et par engagement positif, au moins par sections à la tige allongée (1) et/ou au moins par sections à un insert en forme de cassette (23) pouvant être positionné de manière amovible dans la tige allongée (1),
et **en ce que** le corps de support (7), engagé positivement à la tige allongée (1) ou à l'insert en forme de cassette (23), est prévu pour permettre le verrouillage d'une rotation de l'appareil de verrouillage agencé dans le dispositif de réception (5).

2. Le clou intramédullaire de verrouillage (100) selon la première revendication, où le corps de support (7) est relié de manière amovible à la tige allongée (1) et/ou à l'insert en forme de cassette (23) au moyen d'une liaison rainure-arête ou est partiellement inséré dans une rainure.

3. Le clou intramédullaire de verrouillage (100) selon la revendication 1 ou 2, où le corps de support (7) comprend au moins un rebord, une saillie ou une arête (15) sur sa périphérie extérieure.

4. Le clou intramédullaire de verrouillage (100) selon l'une quelconque des revendications précédentes, où la tige (1) du clou intramédullaire de verrouillage (100) et/ou le ou un insert en forme de cassette (23) comprend/comprennent au moins un orifice (3) pour recevoir l'appareil de verrouillage avec au moins une rainure (17) ou un évidement pour recevoir le corps de support (7).

5. Le clou intramédullaire de verrouillage (100) selon l'une quelconque des revendications précédentes, où le corps de support (7) est agencé entre deux dispositifs de réception (5) successifs ou adjacents.

6. Le clou intramédullaire de verrouillage (100) selon l'une quelconque des revendications précédentes, le corps de support (7) étant en forme de disque et présentant un profil convexe ou concave dans une région centrale.

7. Le clou intramédullaire de verrouillage (100) selon l'une quelconque des revendications précédentes, le corps de support (7) étant de forme annulaire et ayant un profil ouvert dans une région centrale de celui-ci.

8. Le clou intramédullaire de verrouillage (100) selon l'une quelconque des revendications 1 à 5, où le corps de support (7) présente un contour en forme de tige.

9. Le clou intramédullaire de verrouillage (100) selon l'une quelconque des revendications précédentes, où le dispositif de réception (5) est constitué d'un premier matériau, ou comprend un premier matériau, et où le dispositif de réception (5) comprend au moins une partie d'un second matériau différent du premier.

10. Le clou intramédullaire de verrouillage (100) selon l'une quelconque des revendications précédentes, le dispositif de réception (5) étant entièrement ou au moins partiellement revêtu.

11. Le clou intramédullaire de verrouillage (100) selon l'une quelconque des revendications précédentes, le dispositif de réception (5) comprenant au moins un filetage pour recevoir un appareil de verrouillage ayant une partie filetée.

12. Le clou intramédullaire de verrouillage (100) selon l'une quelconque des revendications précédentes, le clou intramédullaire de verrouillage (100) comprenant au moins un appareil d'arrêt (33) pour bloquer au moins un dispositif de réception (5).

13. Le clou intramédullaire de verrouillage (100) selon l'une quelconque des revendications précédentes, le clou intramédullaire de verrouillage (100) comprenant un dispositif excentrique pour bloquer au moins un dispositif de réception (5), le dispositif excentrique générant de préférence une contre-force élastique après application d'une force pour bloquer le dispositif de réception (5) au moyen d'un appareil d'arrêt (33).

14. Le clou intramédullaire de verrouillage (100) selon l'une quelconque des revendications précédentes, où au moins un dispositif de réception (5) comprend au moins un orifice pour le fil de guidage le traversant afin de recevoir un fil de guidage.

15. Le clou intramédullaire de verrouillage (100) selon l'une quelconque des revendications précédentes, où au moins l'un des corps de support (7) comprend au moins un orifice pour le fil de guidage.
